(19) Europäisches Patentamt
European Patent Office
Office européen des brevets

(11) EP 0 914 468 B1

(12) EUROPEAN PATENT SPECIFICATION

(45) Date of publication and mention of the grant of the patent: **28.08.2002 Bulletin 2002/35**

(21) Application number: **97922520.8**

(22) Date of filing: **29.04.1997**

(51) Int Cl.[7]: **C12Q 1/68**

(86) International application number: **PCT/US97/07135**

(87) International publication number: **WO 97/041259 (06.11.1997 Gazette 1997/47)**

(54) **METHOD FOR SEQUENCING OF NUCLEIC ACID POLYMERS**

VERFAHREN ZUR SEQUENZIERUNG VON NUKLEINSÄUREN

PROCEDE DE SEQUENCAGE DE POLYMERES D'ACIDE NUCLEIQUE

(84) Designated Contracting States:
**DE FR GB**

(30) Priority: **01.05.1996 US 640672**
**19.07.1996 US 684498**
**27.02.1997 US 807138**

(43) Date of publication of application:
**12.05.1999 Bulletin 1999/19**

(73) Proprietor: **VISIBLE GENETICS INC.**
**Toronto, Ontario M5G 1Z6 (CA)**

(72) Inventors:
- **LEUSHNER, James**
  **North York, Ontario M5M 4M3 (CA)**
- **HUI, May**
  **Toronto, Ontario M4Y 1G1 (CA)**
- **DUNN, James, M.**
  **Scarborough, Ontario M1K 2S8 (CA)**
- **LARSON, Marina, T.**
  **Yorktown, NY 10598 (US)**
- **LACROIX, Jean-Michel**
  **Etobicoke, Ontario M8Z 5A3 (CA)**
- **SHIPMAN, Robert**
  **Mississauga, Ontario L5N 2N7 (CA)**

(74) Representative:
**Wibbelmann, Jobst, Dr., Dipl.-Chem.**
**Wuesthoff & Wuesthoff,**
**Patent- und Rechtsanwälte,**
**Schweigerstrasse 2**
**81541 München (DE)**

(56) References cited:
**EP-A- 0 655 506**
**WO-A-93/02212**
**WO-A-93/08305**
**WO-A-94/26894**
**WO-A-96/01909**

- **ANALYTICAL BIOCHEMISTRY, vol. 216, no. 1, 1 January 1994, pages 1-14, XP000420647 VENIGALLA B RAO: "DIRECT SEQUENCING OF POLYMERASE CHAIN REACTION-AMPLIFIED DNA"**
- **ANALYTICAL BIOCHEMISTRY, vol. 224, no. 1, 1 January 1995, pages 117-121, XP000486746 WIEMANN S ET AL: "SIMULTANEOUS ON-LINE DNA SEQUENCING ON BOTH STRANDS WITH TWO FLUORESCENT DYES"**
- **NATURE, vol. 376, 31 August 1995, page 796/797 XP000606193 REEVE M A ET AL: "A NOVEL THERMOSTABLE POLYMERASE FOR DNA SEQUENCING" cited in the application**
- **PCR METHODS & APPLICATIONS, vol. 3, no. 5, April 1994, pages S107-S112, XP000606764 KRETZ K ET AL: "CYCLE SEQUENCING"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 914 468 B1

## Description

BACKGROUND OF THE INVENTION

This application relates to DNA sequencing reactions, and in particular to improved sequencing reaction protocols making use of thermally stable polymerase enzymes having enhanced capacity to incorporate chain-terminating nucleotides during chain termination sequencing reactions.

DNA sequencing can be performed in two distinct environments: a research environment in which each procedure is fairly unique and in which the sequence being determined is generally not known prior to completion of the sequence determination; and a diagnostic environment in which the same procedure is repeated on many samples and the sequences being determined are generally known. While the basic procedures used in these two environments can be the same, requirements for speed, cost-effectiveness and low risk of error in the diagnostic environment make many of the techniques actually employed too cumbersome to permit their effective utilization. This has limited the availability of sequencing-based diagnostics, and has indeed led some to question whether sequencing can ever be cost effective for routine diagnostic use.

The ideal DNA sequencing procedure for use in a diagnostic environment would have the following characteristics: (1) it would be able to utilize a DNA-containing sample which had been subjected to only minimal pretreatment to make the DNA accessible for sequencing; (2) it would require combining this sample with only a single reaction mixture, thus reducing risk of error and contamination, and increasing the ease with which the procedure can be automated; and (3) it would require a short amount of time to perform the sequence determination, thus decreasing the marginal costs in terms of equipment and labor for performing the test.

DNA sequencing, whether for research or diagnostics, is generally performed using techniques based on the "chain termination" method described by Sanger et al., *Proc. Nat'l Acad. Sci. (USA)* 74(12): 5463-5467 (1977). Basically, in this process, DNA to be tested is isolated, rendered single stranded, and placed into four vessels. In each vessel are the necessary components to replicate the DNA strand, i.e., a template-dependant DNA polymerase, a short primer molecule complementary to a known region of the DNA to be sequenced, and the standard deoxynucleotide triphosphates (dNTP's) commonly represented by A, C, G and T, in a buffer conducive to hybridization between the primer and the DNA to be sequenced and chain extension of the hybridized primer. In addition, each vessel contains a small quantity of one type (i.e., one species) of dideoxynucleotide triphosphate (ddNTP), e.g. dideoxyadenosine triphosphate (ddA).

In each vessel, the primer hybridizes to a specific site on the isolated DNA. The primers are then extended, one base at a time to form a new nucleic acid polymer complementary to the isolated pieces of DNA. When a dideoxynucleotide triphosphate is incorporated into the extending polymer, this terminates the polymer strand and prevents it from being further extended. Accordingly, in each vessel, a set of extended polymers of specific lengths are formed which are indicative of the positions of the nucleotide corresponding to the dideoxynucleotide in that vessel. These sets of polymers are then evaluated using gel electrophoresis to determine the sequence.

Anal. Biochem. 24, 1995, 117-121 discloses simultaneous sequencing on both strands of ds DNA using one primer for each strand, with each primer being labelled with a different fluorescent dye, i.e. fluorescein and Texas Red EP-A-0655506 and Nature 376 (1995) 796 disclose thermally stable polymerases having modified Incorporation rates.

As Church and Gilbert observed, "in a mammalian cell, the DNA corresponding to any gene sequence is surrounded by DNA corresponding to some million other sequences." "The Genomic Sequencing Technique" in *Medical Genetics: Past*, *Present and Future*, Alan R. Liss, Inc., pp. 17-21, (1991). The same is true, to a greater or lesser extent, of any complex DNA sample, e.g. containing microbial genetic materials, plant genetic materials, complete cDNA libraries etc. In the past, DNA sequencing procedures have dealt with this complexity by adding steps which substantially purify the DNA of interest relative to other DNA species present in the sample. This purification has been accomplished by cloning of the DNA to be sequenced prior to sequencing, or by amplification of a selected portion of the genetic material in a sample to enrich the concentration of a region of interest relative to other DNA. For example, it is possible to amplify a selected portion of a gene using a polymerase chain reaction (PCR) as described in U.S. Patents Nos. 4,683,194,4,683,195 and 4,683,202, which are incorporated herein by reference. This process involves the use of pairs of primers, one for each strand of the duplex DNA, that will hybridize at a site located near a region of interest in a gene. Chain extension polymerization (without a chain terminating nucleotide) is then carried out in repetitive cycles to increase the number of copies of the region of interest many times. The amplified polynucleotides are then separated from the reaction mixture and used as the starting sample for the sequencing reaction. Gelfand et al. have described a thermostable enzyme, "Taq polymerase." derived from the organism *Thermus aqualicus*, which is useful in this amplification process. *(See* US Patent Nos. 4,889,818; 5,352,600 and 5,079,352 which are incorporated herein by reference) Taq polymerase has also been disclosed as useful in sequencing DNA when certain special conditions are met. US Patent No. 5,075,216, incorporated herein by reference.

Improvements to the original technique described by Sanger et al. have included improvements to the enzyme used to extend the primer chain. For example, Tabor et al. have described enzymes such as T7 DNA polymerase which have increased processivity, and increased levels of incorporation of dideoxynucleotides. (*See* US Patent No. 4,795,699 and EP-A-0 386 857, which are incorporated herein by reference). More recently, Reeve et al. have described a thermostable enzyme preparation, called Thermo Sequenase™, with improved qualities for DNA sequencing. *Nature* 376: 796-797 (1995); EP-A-0 655 506, which is incorporated herein by reference. For sequencing, the Thermo Sequenase™ product is used with an amplified DNA sample containing 0.5-2 µg of single stranded DNA (or 0.5 to 5 µg of double stranded DNA) into four aliquots, and combining each aliquot with the Thermo Sequenase™ enzyme preparation, one dideoxynucleotide termination mixture containing one ddNTP and all four dNTP's; and one dye-labeled primer which will hybridize to the DNA to be sequenced. The mixture is placed in a thermocycler and run for 20-30 cycles of annealing, extension and denaturation to produce measurable amounts of dye-labeled extension products of varying lengths which are then evaluated by gel electrophoresis. EP-A-0 655 506 further asserts that Thermo Sequenase™ and similar enzymes can be used for amplification reactions.

Notwithstanding the observations in the art that enzymes useful for amplification can also be used for sequencing, and vice versa, efforts to combine the amplification reaction and the sequencing reaction into a single step have been limited. Ruano and Kidd, *Proc. Nat'l. Acad. Sci. (USA)* 88: 2815-2819 (1991) and U.S. Patent No. 5,427,911, which are incorporated herein by reference, describe a process which they call "coupled amplification and sequencing" (CAS) for sequencing of DNA. In this process, a sample is treated in a first reaction stage with two primers and amplified for a number of cycles to achieve 10,000 to 100,000-fold amplification. A ddNTP is then added during the exponential phase of the amplification reaction, and the reaction is processed for additional thermal cycles to produce chain-terminated sequencing fragments. The CAS process does not achieve the criteria set forth above for an ideal diagnostic assay because it requires an intermediate addition of reagents (the ddNTP reagents). This introduces and opportunity for error or contamination and increases the complexity of any apparatus which would be used for automation.

It is an object of the present invention to provide a method for sequencing of high-complexity DNA samples which is well-suited for use in the diagnostic environment and for automation.

It is a further object of the invention to provide a method for sequencing of DNA which utilizes a DNA-containing sample which had been subjected to only minimal pretreatment to make the DNA accessible for sequencing.

It is still a further object of the invention to provide a method for sequencing of DNA which requires combining a complex DNA-containing sample with only a single reaction mixture, thus reducing risk of error and contamination, and increasing the ease with which the procedure can be automated.

## SUMMARY OF THE INVENTION

The present invention provides a method for sequencing a region of interest in a DNA sample in which a single set of reagents is added to a minimally-treated sample to produce useful sequencing results. The invention is based on the surprising observation and discovery that the addition of a reaction mixture containing the thermostable polymerase Thermo Sequenase™, two primers which bind to complementary strands of a target DNA molecule at sites flanking the region of interest, a mixture of nucleotide triphosphates (A, C, G and T) and one dideoxynucleotide triphosphate to a DNA sample which contains target and non-target DNA in substantially natural abundance, including highly complex DNA samples such as genomic human DNA, and the processing of the combination through multiple cycles of annealing, extension and denaturation results in the production of a mixture which can be loaded directly onto a gel for sequence analysis of the region of interest.

One aspect of the present invention is a method for sequencing a selected region of a target nucleic acid polymer comprising the steps of

(a) combining a natural abundance sample containing the target nucleic acid polymer with a reaction mixture comprising all four types of deoxynucleotide triphosphates, a dideoxynucleotide triphosphate, first and second primers and a thermally stable polymerase enzyme which incorporates dideoxynucleotides into an extending nucleic acid polymer at a rate which is no less than about 0.4 times the rate of incorporation of deoxynucleotides to form a reaction mixture. said first and second primers binding to the sense and antisense strands, respectively, of the target nucleic acid polymer at locations flanking the selected region;

(b) exposing the reaction mixture to a plurality of temperature cycles each of which includes at least a high temperature denaturation phase and a lower temperature extension phase to produce a product mixture comprising sequencing fragments which are terminated by incorporation of the dideoxynucleotide; and

(c) evaluating the product mixture to determine the lengths of the sequencing fragments produced.

## BRIEF DESCRIPTION OF THE DRAWINGS

Fig. 1 illustrates the method of the invention schematically;
Figs. 2A and 2B show a comparison of sequencing runs performed using Thermo Sequenase™ as the polymerase in the method of the invention with results obtained using other thermostable polymerases in a comparative experiment;
Fig. 3 shows the data trace of Fig. 2A in greater detail;
Fig. 4 illustrates a multi-dye embodiment of the invention;
Fig. 5 illustrates a second multi-dye embodiment of the invention;
Figs. 6A and 6B illustrate a third multi-dye embodiment of the invention; and
Figs. 7A and 7B show results for a multi-dye method according to the invention.

## DETAILED DESCRIPTION OF THE INVENTION

The present invention answers the need for a simple and readily-automated sequencing procedure which can be used directly on samples which contain complex mixtures of DNA. To distinguish such mixtures from DNA preparations which have been sequenced in the past, the specification and claims of this application use the term "natural abundance sample" to describe such a mixture. As used herein a "natural abundance sample" is a sample which has been treated to make DNA in the sample accessible for hybridization with oligonucleotide primers, for example by lysis, centrifugation to remove cellular debris and proteolytic digestion to expose the DNA, but which has not been subjected to a preferential purification or amplification step to increase the amount of target DNA relative to non-target DNA present in the initial sample. The term "natural abundance" does not, however, require the presence of all the DNA from the original sample. Thus, a complex sample containing just nuclear DNA, or just mitochondrial DNA or some subfraction of nuclear or mitochondrial DNA obtained by isolation from a tissue sample but not subjected to preferential amplification would be a "natural abundance" sample within the meaning of that term in the specification and claims of this application. The term "natural abundance" would also include a DNA sample prepared by conversion, for example by reverse transcription, of a total mRNA preparation or the genome of an RNA virus to cDNA; DNA isolated from an individual bacterial colony growing on a plate or from an enriched bacterial culture; and a viral DNA preparation where substantially the entire viral genome is isolated. The term "natural abundance" does not encompass a sample in which the isolated DNA is not a complex combination of DNA molecules, and thus would not encompass, for example, a purified plasmid preparation containing only a single species of plasmid.

Natural abundance samples of mammalian DNA can be prepared from fluid samples, e.g., blood or urine or tissue samples by any of a number of techniques, including lysis, centrifugation to remove cellular debris and proteolytic digestion to expose the DNA; salt precipitation or standard SDS-proteinase K-phenol extraction. Natural abundance samples can also be prepared using kits, for example the Gentra Pure Gene DNA Isolation Kit.

The method of the invention utilizes the properties of enzymes like Thermo Sequenase™, namely the ability to incorporate dideoxynucleotides into an extending polynucleotide at a rate which is no less than about 0.4 times the rate of incorporation of deoxynucleotides, to provide a method for the sequencing of a nucleic acid polymer from a natural abundance sample in a single set of thermocycling reactions which can be carried out in a single vessel. Thus, the method of the invention is ideally suited for automation.

Fig. 1 illustrates the fundamental simplicity and elegance of the method of the invention in flow chart form. As shown in Fig. 1, a sample containing a target nucleic acid polymer which includes a region to be sequenced is combined with a reaction mixture containing two primers, a mixture of dNTP's, a chain terminating nucleotide triphosphate, i.e., a dideoxynucleotide triphosphate, and a thermostable polymerase with a high affinity for ddNTP incorporation in a buffer suitable for hybridization and template-dependant polymerization. The mixture is processed for a number of thermal cycles sufficient to produce detectable amounts of sequencing fragments, generally from 20 to 50 cycles. During each cycle, the primers each anneal to the respective strand of target DNA present in the sample, and primer chain extension using the polymerase enzymes and the nucleotide triphosphate feedstocks proceeds until terminated by incorporation of a chain-terminating nucleotide triphosphate. This results in the production of sequencing fragments comparable to those generated in a conventional sequencing reaction. Analysis of these fragments provides information concerning the sequence of the selected region of the target DNA. Those extension products which are not terminated prior to reaching the region complementary to the other primer can serve as template for generation of sequencing fragments in later cycles, although this generally occurs to a very small extent. Finally, the product mixture containing dideoxy-terminated fragments is loaded onto an electrophoresis gel for analysis of the positions of the base corresponding to the chain-terminating nucleotide triphosphate with in the target nucleic acid polymer.

The operation of the invention can be understood in the context of a hypothetical 200 nt DNA fragment having equal amounts of each base. This means that there will be 50 potential truncation events during the cycle. For each cycle, some of the products would be full length (and thus able to hybridize with one of the two primers to produce more sequencing fragments) and some would be truncated at the points where the ddNTP was added. If each of these truncation events has a statistical likelihood of occurring 1 time in 500 as a result of the relative concentration of ddNTP compared to dNTP and the relative incorporation by the enzyme, then overall a truncation product will occur in slightly less than ten percent of the reactions. Table 1 shows the relative amounts of full-length and chain-termination products theoretically formed after 10, 20 and 30 cycles of a reaction according to the invention using this 200 nt polynucleotide assuming various ratios of truncated to full-length product.

TABLE 1

| Cycles | truncation ratio =0.1 | | truncation ratio =0.3 | | truncation ration = 0.5 | |
|---|---|---|---|---|---|---|
| | truncated | full-length | truncated | full-length | truncated | full-length |
| 10 | 32 | 613 | 86 | 202 | 57 | 57 |
| 20 | 41,000 | 376,000 | 17,400 | 40,462 | 3,300 | 3,300 |
| 30 | 25.6 X $10^6$ | 230 X $10^6$ | 3.5 X $10^6$ | 8.2 X $10^6$ | 190,000 | 190,000 |

The absolute and relative amounts of nucleotide triphosphates and chain-terminating nucleotide triphosphates may be optimized for the particular enzyme employed. In actual practice, it has been found that useful results are obtained with Thermo Sequenase™ when the reaction is run for 35 to 45 cycles, using a dideoxy:deoxy mole ratio of 1:100 to 1:300. In general, each nucleotide triphosphate will be included in the reaction mixture at concentrations of from 250 μM to 1.5 mM, and the chain-terminating nucleotide triphosphate will be included at a level of from 0.5 μM to 30 μM to produce compositions in which the mole ratio of the chain terminating nucleotide triphosphate to the corresponding nucleotide triphosphate is from 1:50 to 1:1000, preferably from 1:100 to 1:500. This will result in incorporation of a chain-terminating nucleotide triphosphate into from 30 to almost 100 percent of the extending polymer chains formed during the thermal cycling of the reaction mixture.

A key factor in successfully performing the method of the invention is the utilization of Thermo Sequenase™ or a comparable enzyme as the thermostable polymerase in the reaction mixture. Such enzymes are characterized by a high affinity for incorporating dideoxynucleotides into the extending nucleotide chain. In general, for purposes of the present invention, the polymerase used should be one which incorporates dideoxynucleotides into an extending nucleic acid polymer at a rate which is no less than about 0.4 times the rate of incorporation of deoxynucleotides. Thermo Sequenase™ is known to favor the incorporation of dideoxynucleotides, and is suitable for use in the invention.. Tabor et al. have also described enzymes having increased processivity and high and increased levels of incorporation of dideoxynucleotides. (See EP 0 655 506). Roche sells a polymerase under the trademark TAQ-FS which meets these criteria as well.

Figs. 2A and 2B and Fig. 3 illustrate the importance of this characteristic of the polymerase enzyme employed. Figs. 2A and 3 shows a sequencing data trace for an actual heterozygous patient sample of natural abundance DNA which was obtained using Thermo Sequenase™ and primers flanking exon 2 of the Von Hippel-Lindau gene in a process according to the invention. Large, well-defined peaks corresponding to the termination fragments were obtained which made sequence evaluation of the sample very straightforward. In addition, the peaks for homozygous peaks are all approximately the same size, and are readily distinguishable from peaks for bases at heterozygous locations. This result was obtained performing the test in a single reaction vessel, with a single unaugmented reaction mixture, in a total of 45 thermal cycles. Comparable results could be obtained using fewer reaction cycles, for example 35 cycles as shown in Example 1 herein.

In contrast, Fig. 2B shows the trace obtained when a combination of Vent and Sequitherm™ were used instead of Thermo Sequenase™ for a total of 45 thermal cycles. In this trace, the peaks for the termination fragments are much smaller and less well defined. Furthermore, the peaks are quite variable in height and did not permit identification of heterozygous peaks based on peak height. Performing the same experiment using Taq polymerase alone resulted in a data trace that contained no usable peaks.

In the method of the invention, a natural abundance sample containing, or suspected to contain, a target DNA sequence is combined in a reaction mixture with an appropriate polymerase, all four types of deoxynucleotide triphosphates, a dideoxynucleotide triphosphate, and first and second primers. The primers used in the method of the present invention can be any pair of primers which hybridize with the sense and antisense strands of the target DNA flanking a selected region that is to be sequenced, and which do not both hybridize to neighboring locations in human DNA or other DNA potentially found in the sample. As used herein, the term "flanking" will be understood to mean the positioning of primers at the 5'-ends of the selected region on each DNA strand, such that extension of the primers leads to replication of the region between the primers. The primers are preferably selected such that the primer pair flanks a region that is about 500 bp or less, although primers spanning larger regions of DNA can be utilized with adjustments to the sequencing mixture (generally an increase in the relative amount of deoxynucleotide triphosphates) to increase the amount of longer sequencing fragments produced.

Primers can be selected to hybridize with highly conserved regions which are the same in all variants of the target DNA or can be prepared as degenerate primers to take known sequence variations at the primer site into account. Thus, the first and second primers of the invention may each be a discrete oligonucleotide species, or may be a set of oligonucleotide primers with similar but not identical sequences.

One or both of the primers may be labeled with a detectable label at the 5'-end thereof, particularly a fluorescent label such as fluorescein or a cyanine dye such as Cy 5.5. If labels are used on both primers, the labels selected should be spectroscopically-distinct, i.e., they should have either a different excitation spectrum or a different emission spectrum such that one primer can be distinguished from the other. When both primers are labeled with different detectable labels, for example with two different fluorophores as in the process described by Wiemann et al., "Simultaneous On-Line DNA Sequencing on Both Stands with Two Fluorescent Dyes," *Anal. Biochem* 224: 117-121 (1995), the sequence of both strands of the sample can be determined in a single reaction.

The nucleotide triphosphate feedstock mixture is a standard mixture of the four conventional deoxynucleotide bases (A, C, G and T) in a buffer suitable for template-dependent primer extension with the enzyme employed. As will be appreciated by persons skilled in the art, the specific concentrations of the nucleotide triphosphates and the nature of the buffer will vary depending on the enzyme employed. Standard buffers and reagent concentrations for various known polymerase enzymes may be employed in the invention.

The reaction mixture used in the present invention also includes at least one type (or one species) of chain-terminating nucleotide triphosphate. Separate reactions for the four different types of bases may be run either concurrently or successively. Running all four bases concurrently comports with conventional sequencing practice. However, a preferred embodiment of the present invention combines the single vessel methodology of this application with "single track sequencing" which is described in commonly assigned US Patent Application No. 08/577,858. In single track sequencing, the determination of the positions of only one (or in any event less than 4) nucleotide(s) of a target sequence is frequently sufficient to establish the presence of and determine the qualitative nature of a target microorganism by providing a fingerprint or bar-code of the target sequence that may be sufficient to distinguish it from all other known varieties of the sequence. Throughput is increased by reducing the number of reactions and electrophoresis runs required to identify a sequence. By selection of the order of bases tested, and intermediate analysis, it may be unnecessary to run all four bases to determine the presence and specific qualitative nature of any target microorganism present in the sample.

The present method can be used in combination with any type of detection system that is compatible with the label employed on the primers. For example, a Pharmacia A.L.F. sequencer may be employed when fluorescein-labeled primers are used, while a Visible Genetics MicroGene Blaster is appropriate when the label used is Cy5.5. When multiple labels are used, the sample can be processed on multiple instruments, or it can be evaluated on an instrument which is capable of detecting signals from multiple labels. An example of such an instrument is the Prism 377 Sequencer (Applied Biosystems Inc.) which detects and distinguishes between 4 dyes in a single lane. Spectroscopically distinguishable dyes which are recognized by the Prism 377 are the FAM, ROX, TAMRA and JOE dyes known in the art.

The possibility of multi-dye detection leads to a wide range of applications for the invention which lead to improved accuracy in sequencing and to improved instrumental throughput. For example, Fig. 4 illustrates a method for obtaining both the forward and reverse sequences of a target nucleic acid sequence using two primers, each with a spectroscopically-distinguishable label. The natural abundance sample is mixed with forward and reverse primers, each with a distinguishable label (1 and 2). The reaction is performed with four termination reactions, one each for A, C, G and T. Each reaction is loaded into a single well of an automated sequencing instrument that detects and distinguishes at least the two labels employed. The results detected from label 1 are combined to give the forward sequence. The results detected from label 2 are combined to give the reverse sequence. The two sequences can be used to check each other and correct any ambiguities in base calling. In addition, the opposite sequence can be used to confirm sequence proximal to a primer which is found empirically to be difficult to determine on commercially available automated DNA sequencers.

Fig. 5 demonstrates the use of multiple labels in a different format. In this case, assuming the instrument can distinguish between 4 labels, 4 genes or gene fragments in a single sample can be sequenced contemporaneously. 4 pairs of primers are added to patient sample genomic DNA. Each pair is specific for a different target, possibly 4 exons of the same gene of interest (P, Q, R and S). One member of each pair is conjugated to a detectable label (1,2,3, or 4) and each label is distinguishable from the others. This mixture is divided into four termination reaction tubes, one each for ddA, ddC, ddG and ddT, and thermally processed. Termination reaction products are loaded in one lane, thus employing the method as disclosed in US Patent Application Serial No. 08/634,284, assigned to the assignee of the instant invention and incorporated herein by reference. The results from each label are combined to give the sequence of the exon for which that primer is specific.

Another embodiment takes advantage of the fact that a single ddA termination reaction identifies the A nucleotides from each strand, Fig. 6A, thus identifying the complementary base (in this case T) in the opposite strand. (i.e. the A termination sites on the opposite strand correspond to T nucleotide sites in the first strand). The complementary base can be located in the "missing" sites of the opposite strand. Note that sequence from the opposite strand must be inverted before it can be added in to the missing sites because it starts at the opposite end of the target gene and chain extension is in the opposite direction.

In Fig. 6B, a second termination reaction for ddC is added. This allows identification of C and its complement G in each strand. When these results are added to the first reaction, a full DNA sequence is obtained. Thus on the basis of 2 termination reactions employing one ddNTP chain terminator each, the full 4 lane sequence of a gene can be obtained.

The base-calling and compiling of sequences illustrated in Fig. 6A and 6B can be facilitated using GeneObjects software (Visible Genetics Inc., Toronto) and employing techniques disclosed in US Patent Application Nos. 08/497,202 and 08/670,534, incorporated herein by reference.

The method of the present invention is advantageously applied in many contexts including: (1) detection of mutations, particularly mutations of medical significance, in samples derived from a human patient, animal, plant or microorganism; (2) determination of HLA type ancillary to transplant procedures; (3) detection and identification of microorganisms, particularly pathogenic microorganisms, in a sample; and (4) *in-situ* sequencing reactions to produce sequencing fragments within a histological specimen which are then removed from a selected location on the tissue preparation and loaded onto a gel for sequence analysis. This latter approach is particularly useful for evaluation of archived samples in retrospective studies where the outcome of a disease condition is known, but the potentially causative mutation is not. This method can be used with labeled primers for single base sequencing (or multiple-base sequencing using multiple tissue samples).

The basic method of the invention can also be enhanced by various modifications without departing from the scope of the present invention. For example, improvements in reproducibility and sensitivity can be obtained by using a combination of an enzyme having a high affinity for incorporation of dideoxynucleotide triphosphates into the extending polymer, e.g., Thermo Sequenase™, and one having a low affinity for incorporation of dideoxynucleotide triphosphates into the extending polymer, e.g., Taq polymerase, under conditions where both enzymes are actively catalyzing template-dependent primer extension polymerization. As noted above, the high affinity enzyme produces almost entirely termination products, with very few of the polymers actually being extended to full length. On the other hand, the low affinity enzyme produces almost exclusively full length product, with relatively few termination products. Addition of the low affinity enzyme to the reaction mixture increases the sensitivity of the method by producing more full length material to be sequenced without increasing the processing time or adding processing steps. The increase in sensitivity can be controlled by varying the ratio of high affinity to low affinity enzyme present in the mixture.

It will be noted, however, that including of low affinity enzyme to produce full length product will also result in the formation of a very intense labeled full-length product peak. This peak may make analysis of the bases near the end of the sequence difficult. To obtain the benefits of increased sensitivity while making less full length product, it may be desirable to utilize a low affinity enzyme which is more thermolabile than Taq polymerase, such that the low affinity enzyme is essentially inactivated by the end of the first 15 to 25 cycles. This would allow the production of longer fragments early in the assay and the generation of more terminated fragments late in the assay.

The reaction mixture of the invention may also incorporate other additives which enhance the formation of sequencing fragments. For example, a product called TaqStart™ Antibody is a monoclonal antibody which binds to and blocks the activities of Taq polymerase. This antibody is added to PCR reactions using Taq polymerase to block enzyme activity during set-up at ambient temperature to prevent or reduce the formation of non-specific amplification products. TaqStart™ Antibody can be used in the present invention with Thermo Sequenase™ to reduce nonspecific primer extension reactions.

Other materials which can be used in the reaction mixture of the invention are uracil-DNA glycosylases and corresponding unconventional nucleotides as described in US Patent No. 5,418,149, incorporated herein by reference, to reduce non-specific product formation. Roche sells a product under the trademark AMPERASE™ which can be used conveniently for this purpose. The method of the invention may also be used in conjunction with Johnson & Johnson techniques known as "PCR IN A POUCH" which is described in US patent No. 5,460,780 incorporated herein by reference.

EXAMPLE 1

A 175 ng sample of genomic DNA is prepared from a patient blood sample using the Gentra Pure Gene™ DNA isolation kit. Briefly, in this procedure the blood cells were lysed, centrifuged to recover the lysed white blood cells and mixed with proteinase K. Protein is then separated from the sample by precipitation and the remaining nucleic acids are precipitated and collected. The resulting genomic DNA preparation was combined with two primers flanking exon 2 of the VHL gene.

5' primer - labeled with the fluorophore Cy5;
GGCTCTTTAA CAACCTTT [SEQ ID No.: 1]

3' primer - unlabeled;
GGGCTTAATT TTTCAAGTGG TC [SEQ ID No.: 2]

The reaction mixture employed had the following composition:

| | final amt. | final Vol. |
|---|---|---|
| DNA genomic | 175 ng | 3.5 ul |
| 5' Primer 1pM/ul | 3 pMol | 3.0 ul |
| 3' Primer) 7.5pM/ul | 23 pMol | 3.0 ul |
| DMSO 100% | | 1.5 ul |
| Thermo Sequenase Reaction Buffer | | 2.0 ul |
| Thermo Sequenase Enzyme 32U/ul | 6.4 U | 0.2 ul |
| | Total | 13.2 ul |

3 ul aliquots of the reaction mixture were placed into each of 4 tubes containing 3 ul of one of the following termination mixes: A, C, G or T (dNTP/ddNTP; 100:1 ratio; 750 µM: 7.5 µM)after which the mixture was layered with oil.

The mixture was then processed in PTC 100 Thermocycler at follows:

denature

95°C 120 sec

35 cycles

95°C 50 sec
52°C 30 sec
70°C 60 sec

finish

70°C 120 sec
6°C soak

6 ul dye/stop solution was then added to each tube to a final volume of 12 ul. A 2 ul sample was then loaded onto a thin polyacrylamide gel and analyzed in a MicroGene Blaster sequencer (Visible Genetics Inc, Toronto, Canada). The result was a clean, easily interpreted sequencing ladder.

EXAMPLE 2

A 500 ng sample of natural abundance DNA prepared from a patient sample using a standard SDS-Proteinase K-phenol extraction was combined with the same two primers as in Example 1 for preparation of sequencing fragments from exon 2 of the VHL gene. The reaction mixture employed had the following composition:

| | final amt. | final vol. |
|---|---|---|
| DNA genomic | 500 ng | 1.0 ul ul |

(continued)

| | final amt. | final vol. |
|---|---|---|
| 5' Primer) 3 pMol | | 3.0 ul |
| 3' Primer 3 pMol | | 3.0 ul |
| DMSO 100% | | 1.5 ul |
| Thermo Sequenase Reaction Buffer | | 2.0 ul |
| Thermo Sequenase Enzyme 32U/ul | 6.4 U | 0.2 ul |
| distilled water | | 3.0 ul |
| | Total Volume | 13.2 ul |

3 ul aliquots of the reaction mixture were placed into each of 4 tubes containing 3 ul of one of the following termination mixes: A, C, G or T (dNTP/ddNTP; 100:1 ratio; 750 µM: 7.5 µM) after which the mixture was layered with oil.

The mixture was then processed in PTC 100 Thermocycler at follows:

denature

94°C 120 sec

45 cycles

94°C 20 sec
52°C 20 sec
72°C 20 sec

finish

72°C 150 sec
6°C soak

6 ul dye/stop solution was then added to each tube to a final volume of 12 ul. A I ul sample was then loaded onto a thin polyacrylamide gel and analyzed in a MicroGene Blaster sequencer (Visible Genetics Inc, Toronto, Canada).

For comparison, a sample of the same natural abundance DNA was treated in a similar reaction using a mixture of Vent Enzyme and Sequitherm Enzyme as follows:

| | final amt. | final vol. |
|---|---|---|
| DNA genomic | 1 ug | 8.0 ul |
| 5' Primer (Cy5.5 labeled) | 12.5 pMol | 12.5 ul |
| 3' Primer (unlabeled) | 12.5 pMol | 12.5 ul |
| Triton X-100 20% | | 25 ul |
| 1 mM each dNTP | | 4.0 ul |
| 10X Vent Buffer | | 5.0 ul |
| 10X Sequitherm Buffer | | 5.0 ul |
| Vent Enzyme 2U/ul | | 2.0 ul |
| Sequitherm Enzyme 5 U/ul | | 2.0 ul |
| distilled water | | 6.5 ul |
| | Total Volume | 82.5 ul |

20 ul of reaction mixture of aliquoted into each of 4 tubes containing 5 ul of one of the following ddNTP in water:

| | |
|---|---|
| ddATP | 850 uM |
| ddCTP | 500 uM |
| ddGTP | 100 uM |
| ddTTP | 1700 uM |

and layered with oil. These mixtures were processed in a PTC 100 thermocycler as follows:

denature

94° 90 sec

45 cycles

94° 20 sec
52° 20 sec
72° 20 sec

finish

72° 150 sec
6° soak

25 ul of dye/stop solution as then added to each tube to a final volume of 50 ul. 2 ul aliquots of this solution were loaded onto a MicroGene Blaster sequencer for analysis.

The sequencing traces taken for these experiments are shown in Figs. 2A-2B and 3. Figs. 2A and Fig. 3 show the result for the Thermo Sequenase™ runs according to the invention is vastly superior to the comparative tests even though a smaller volume of initial DNA was used. In particular, not only are the peaks more detectable, the peaks in the Thermo Sequenase™ run are also correctly reflect the fact that the sample was a heterozygote, providing peaks of substantially uniform size to reflect one versus two bases. This makes analysis of the results much easier. Thus, these experiments demonstrate the surprising characteristics of the method of the invention.

EXAMPLE 3

For comparison to the method of the invention, an experiment was conducted in which the ability of Taq Polymerase to produce usable sequencing fragments directly from natural abundance DNA was tested. No sequence information could be obtained using Taq polymerase alone.

EXAMPLE 4

The sequence of the p53 gene in a patient sample is obtained, according to the method of the invention, as follows. Patient sample DNA is obtained from a tumor biopsy sample according to a standard method known in the art. A Pre-Reaction Mixture is prepared:

| | |
|---|---|
| Patient sample genomic DNA (100 ng/ul) | 3.0 ul |
| 1:10 Diluted Thermo Sequenase Enzyme (Amersham) | 2.0 ul |
| 10X Enzyme Reaction Buffer (Amersham) | 2.0 ul |
| Primer A (10 uM) | 0.6 ul |
| Primer B (10 uM) | 0.6 ul |
| DMSO | 1.3 ul |
| ddH2O | 3.5 ul |
| | 13.0 ul |

The primers employed as Primer A and Primer B depend on the specific p53 exon sequence desired. A non-exclusive list of primers useful in practicing the invention are set out in Table 2. At least one of the primers must be labeled with a detectable label if used in a fluorescence-based automated DNA sequencer.

Separate termination mixtures are also prepared using a ratio of deoxynucleotide triphosphates (dNTPs) to dideoxynucleotide triphosphates (ddNTPs) of 300 : 1.

A Termination Mix: 750 uM each dNTP (dATP, dCTP, dGTP, dTTP); 2.5 uM ddATP
C Termination Mix: 750 uM each dNTP (dATP, dCTP, dGTP, dTTP); 2.5 uM ddCTP
G Termination Mix: 750 uM each dNTP (dATP, dCTP, dGTP, dTTP); 2.5 uM ddGTP
T Termination Mix: 750 uM each dNTP (dATP, dCTP, dGTP, dTTP); 2.5 uM ddTTP

3 ul of the Pre-Reaction Mixture is added to 3 ul of each Termination Mix, and mixed well. This Reaction Mixture is then treated to the following temperature cycles in an automated thermocycler (such as the MJ Research PTC-100 Programmable Thermal Controller):

94°C 5 min

then 40 cycles of

94°C 30 sec
60°C 30 sec
70°C 60 sec

and a final extension reaction of 5 mins at 70°C. The Reaction Mixture is then placed on ice. 6 ul of STOP/Loading buffer (100% formamide; colored dye) is added and mixed. 1.5 ul of the final mixture is loaded in a single well of a MicroGene Blaster automated DNA sequencer (Visible Genetics Inc., Toronto). The reaction products are separated by electrophoresis and detected. GeneObjects software (Visible Genetics Inc.) is used to analyze the results and present the sequence. The results are reported to the patient file.

TABLE 2

(primer and exon locations are given using nucleotide numbers in the sequence of p53 (GeneBank Accession No. 54156) submitted by Chumakov et al.

| Exon | Sequence | Primer Location | Fragment Size | Exon Location | Primer Conc (umol/L) |
|---|---|---|---|---|---|
| 1 | CGGATTACTT GCCCTTACTT GTCA [SEQ ID 3 ] | 711 | 331 | 843-949 | 0.4 |
| | CCCCAGCCCC AGCGATTTT [SEQ ID 4 ] | 1041 | | | |
| 2 | CCAGGGTTGG AAGCGTCTC [SEQ ID 5] | 11641 | 259 | 11689-11790 | 0.9 |
| | GACAAGAGCA GAAAGTCAGTCC [SEQ ID 6] | 11899 | | | |
| 3 | CATGGGACTG ACTTTCTGCT [SEQ ID 7] | 11874 | 141 | 11906-11927 | 0.8 |
| | ATGGGTGAAA AGAGCAGT [SEQ ID 8] | 12014 | | | |
| 4 | CTGGTCCTCT GACTGCTCTT TTCA [SEQ ID 9] | 11986 | 382 | 12021-12299 | 0.48 |
| | AAAGAAATGC AGGGGGATAC GG [SEQ ID 10] | 12367 | | | |
| 5 | TGTTCACTTG TGCCCTGACT [SEQ ID 11] | 13005 | 268 | 13055-13432 | 0.2 |
| | CAGCCCTGTC GTCTCTCCAG [SEQ ID 12] | 13272 | | | |
| 6 | CTGGGGCTGG AGAGACGACA [SEQ ID 13] | 13247 | 274 | 13320-13432 | 0.14 |
| | GGAGGGCCAC TGACAACCA [SEQ ID 14] | 13493 | | | |

| TABLE 2 (primer and exon locations are given using nucleotide numbers in the sequence of p53 (GeneBank Accession No. 54156) submitted by Chumakov et al. | | | | | |
|---|---|---|---|---|---|
| Exon | Sequence | Primer Location | Fragment Size | Exon Location | Primer Conc (umol/L) |
| 7 | CTCCCCTGCT TGCCACA [SEQ ID 15] | 13933 | 245 | 14000-14109 | 0.4 |
| | GGGTCAGCGG CAAGCAGAGG [SEQ ID 16] | 14177 | | | |
| 8 | GACAAGGGTG GTTGGGAGTA GATG [SEQ ID 17] | 14350 | 320 | 14452-14588 | 0.2 |
| | GCAAGGAAAG GTGATAAAAG TGAA [SEQ ID 18] | 14669 | | | |
| 9 | GCGGTGGAGG AGACCAAGG [SEQ ID 19] | 14609 | 209 | 14681-14754 | 0.1 |
| | AACGGCATTT TGAGTGTTAG AC [SEQ ID 20] | 14817 | | | |
| 10 | TGATCCGTCA TAAAGTCAAA CAA [SEQ ID 21] | 17477 | 390 | 17572-17678 | 0.3 |
| | GTGGAGGCAA GAATGTGGTT A [SEQ ID 22] | 17866 | | | |
| 11 | GGCACAGACC CTCTCACTCA T [SEQ ID 23] | 18540 | 256 | 18599-18876 | 0.4 |
| | TGCTTCTGAC GCACACCTAT T [SEQ ID 24] | 18795 | | | |

EXAMPLE 5

The DNA sequence of exon 2 of the VHL gene was sequenced from a human DNA sample using the method of the invention as follows.

A natural abundance sample is prepared from a human patient blood sample using the Gentra Pure Gene DNA isolation kit according to the manufacturers instructions. Briefly, in the procedure, the blood cells were lysed, centrifuged to recover the lysed white blood cells and mixed with proteinase K. Protein is then separated from the sample by precipitation and the remaining nucleic acids are precipitated and collected.

| | final amt. | final vol. |
|---|---|---|
| DNA | 175 ng | 3.5 ul |
| 5' Primer (Cy5.5 labeled) 1pmol/ul | 3 pmol | 3.0 ul |
| 3' Primer (Fluorescein labeled) 1pmol/ul | 3 pmol | 3.0 ul |
| DMSO 100% | | 1.5 ul |
| 10X ThermoSequenase Reaction Buffer | (Amersham) | 2.0 ul |
| ThermoSequenase Enzyme 32U/ul | 6.4 U | 0.2 ul |
| | | 13.2 ul |

5' primer

5'- GGCTCTTTAA CAACCTTT-3' [SEQ. ID No.: 1] (Cy5.5 labeled)

3' primer

5'-GGGCTTAATT TTTCAAGTGG TC - 3' [SEQ. ID No.: 2] (Fluorescein labeled)

The fluorescent label indicated is conjugated to the 5' end of the primer oligonucleotide by means known in the art. Briefly, this may include phosphoramidite technology commonly employed on automated DNA synthesizers, or a two stage reaction where an amino linker is added to the 5' end of the primer oligonucleotide and condensed with a dye-ester conjugate. The fluorescent dye is selected according to the requirements of the detection device employed.

3 ul aliquots of the reaction mixture were placed into each of 4 tubes containing 3 ul of one of the following termination mixes containing all 4 dNTPs and one of the following ddNTPs: A, C, G or T (dNTP/ddNTP = 100:1 ratio; 750 uM: 7.5 uM) after which the mixture was layered with oil. The mixture was then processed in a PTC 100 Thermocycler as follows:

denature

95°C 120 sec

35 cycles

95 °C 50 sec
52°C 30 sec
70°C 60 sec

finish

70°C 120 sec
6°C soak

6 ul dye/stop solution was then added to each tube to make a final volume of 12 ul. 2ul of final mixture was loaded on a lane of the MicroGene Blaster. 7ul of final mixture was loaded on a lane of an ALF automated Sequencer (Pharmacia). Electrophoresis was performed and the separated reaction products were detected, recorded and evaluated. Figs. 7A and B show the results obtained with the Microgene Blaster which detects the Cy5.5-labeled product and the A.L.F. which detects the fluorescein-labeled product, respectively.

EXAMPLE 6

A human natural abundance DNA sample is prepared from a patient blood sample using the Gentra Pure Gene DNA isolation kit according to the manufacturers instructions. Briefly, in the procedure, the blood cells were lysed, centrifuged to recover the lysed white blood cells and mixed with proteinase K. Protein is then separated from the sample by precipitation and the remaining nucleic acids are precipitated and collected.

The natural abundance sample is combined in a reaction mixture for characterization of the HLA type of the sample as follows:

| | final amt. | final vol. |
|---|---|---|
| DNA (natural abundance) | 175 ng | 3.5 ul |
| 5' Primer (Cy5.5 labeled) 1pmol/ul | 3 pmol | 3.0 ul |
| 3' Primer (Fluorescein labeled) 1pmol/ul | 3 pmol | 3.0 ul |
| DMSO 100% | | 1.5 ul |
| 10X Thermo Sequenase Reaction Buffer (Amersham) | | 2.0 ul |

(continued)

| | | final amt. | final vol. |
|---|---|---|---|
| Thermo Sequenase Enzyme | 32U/ul | 6.4 U | 0.2 ul |
| | | | 13.2 ul |

The fluorescent label indicated is conjugated to the 5' end of the primer oligonucleotide by means known in the art. Briefly, this may include phosphoramidite technology commonly employed on automated DNA synthesizers, or a two stage reaction where an amino linker is added to the 5' end of the primer oligonucleotide and condensed with a dye-ester conjugate. The fluorescent dye is selected according to the requirements of the detection device employed.

HLA-A

**EXON2**

5' Primer

vgiawsp1 GCGCCGGGAGGAGGGTC [SEQ ID 25]

3' Primer

vgiawsp2 GTCGTGACCTGCGCCCC [SEQ ID 26]

**EXON3**

5' Primer

vgiawsp3 GGGCGGGGCGGGGCTCGGG [SEQ ID 27]

3' Primer

vgiawsp4 CGGGAGATCTACAGGCGATCAGG [SEQ ID 28]

HLA-B

**EXON2**

5' Primer

vgibwsp3 TCCCACTCCATGAGGTAT [SEQ ID 29]

3' Primer

vgibwsp4 GTCGTGACCTGCGCCCC [SEQ ID 30]

**EXON3**

5' Primer

vgibwsp5 GGGCGGGGCGGGGCTCGGG [SEQ ID 31]

3' Primer

vgibwsp6 GAAGGCTCCCCACTGCCC [SEQ ID 32]

The primer pair for a specific HLA gene may be selected from the following non-exclusive list:

HLA-C

**EXON2**

5' Primer

vgicwsp3 GGAGGGTCGGGCGGGTCT [SEQ ID 33]

3' Primer

vgicwsp4 GTCGTGACCTGCGCCCC [SEQ ID 34]

**EXON3**

5' Primer

vgicwsp5 GACCGCGGGGGCGGGGCCA [SEQ ID 35]

GACCACGGGGGCGGGGCCA [SEQ ID 36]

3' Primer

vgicwsp6 GAGGCTCCCCACTGCCC [SEQ ID 37]

3 ul aliquots of the reaction mixture were placed into each of 4 tubes containing 3 ul of one of the following termination mixes containing all 4 dNTPs and one of the following ddNTPs: A, C, G or T (dNTP/ddNTP = 100:1 ratio; 750 uM: 7.5 uM) after which the mixture was layered with oil. The mixture was then processed in a PTC 100 Thermocycler as follows:

denature

95°C 120 sec

35 cycles

95°C 50 sec
52°C 30 sec
70°C 60 sec

finish

70°C 120 sec
6°C soak

6 ul dye/stop solution was then added to each tube to make a final volume of 12 ul. 2ul of final mixture was loaded on a lane of the MicroGene Blaster. 7ul of final mixture was loaded on a lane of an ALF automated Sequencer (Pharmacia). Electrophoresis was performed and the separated reaction products were detected, recorded and evaluated.

The time saving of a single sequencing reaction of this type as compared to previously available sequencing methods is illustrated in Table 3.

TABLE 3

| Step | Prior Art Kit | Method of the Invention |
|---|---|---|
| PCR of natural abundance DNA | 2.5 hours | 0 |
| Purify Amplicon with Dynal Beads (optional) | 1 hour | 0 |
| Sequencing Reactions | 2.5 hours | 2 hours |
| Total | 5 to 6 hours | 2 hours |

EXAMPLE 7

The presence of the sexually transmitted disease pathogen *Chlamydia trachomatis* in a patient sample is detected according to the method of the invention as follows.

Urine samples from patients suspected of carrying a sexually transmitted disease pathogen are prepared for sequence- based diagnosis as follows. 100 ul of first void urine are deposited in a sterile microcentrifuge tube. The tube is centrifuged at 12,000 x g for 20 min; the supernatant is removed. 100 ul of Lysis Solution (Proteinase K @ 100 g/ml; 1% Tween 20) is added to the bacterial pellet and incubated 1 h at 55°C, or 18 h at room temperature. After a final incubation at 95 °C for 10 minutes, 200 ul of Geneclean II glass milk is added, according to the manufacturer's instructions. (Bio 101, Inc) DNA is eluted in 10 ul of double distilled $H_2O$. (A lysis solution control may be prepared if desired, by adding the lysis solution to a sterile tube (a tube without any urine pellet), and treating this tube like the others.)

The sample natural abundance DNA is then treated according to the method of the invention with a pair of primers and reagents to identify the sequence of a *C. trachomatis* gene present in the sample, if any. A suitable *C. trachomatis* specific target for sequencing is the cryptic plasmid. Primers that may be used are

| Name | Sequence | |
|---|---|---|
| KL1: | TCCGGAGCGA GTTACGAAGA | [SEQ ID NO: 38] |
| KL2: | ATTCAATGCC CGGGATTGGT | [SEQ ID NO: 39] |

These sequencing primers were employed previously for PCR amplification reactions, but not sequencing (Mahony et al., "Confirmatory polymerase chain reaction testing for Chlamydia trachomatis in first void urine from asymptomatic and symptomatic men" *J. Clin Microbiol.* 30:2241-2245 (1992)).

Either primer may be labeled at the 5'-end with a detectable label such as a Cy5.5 fluorophore. If both primers are labeled, they should be distinguishable. Labels are selected on the basis of the instrument employed for detection. Labeling reactions are performed according to methods well known in the art, such as amidite labeling or dye-ester condensation.

The sequencing reaction mixture is prepared by combining 2.5 ul of the prepared DNA sample, 0.67 ul of 10 uM primer KL1 (labeled with *Cy5.5),* 0.45 ul of KL2 primer at 10 uM, 2 ul of THERMO SEQUENASE reaction buffer (250 mM Tris-HCl pH 9.0 @ 25°C, 39 mM $MgCl_2$), 2 ul of THERMO SEQUENASE enzyme (Amersham Life Sciences) diluted 1/10 in the dilution buffer provided with the enzyme and 5.38 ul of double distilled $H_2O$. The final volume is 13 ul.

3 ul of the sequencing reaction mixture is placed in each of 4 clean tubes and covered with one drop of mineral oil (Sigma Chemical Co., Cat # M-5904). The tube is placed in a PTC-100 thermal cycler (M.J. Research, Maine) and heated for 3 min at 94°C, then cooled to 85°C. One of the following termination mixtures are then added to each of the 4 tubes:

- 3 ul of dNTP:ddATP (1 mM each dNTP, 3.3 uM ddATP) in tube A.
- 3 ul of dNTP:ddCTP (1 mM each dNTP, 3.3 uM ddCTP) in tube C.
- 3 ul of dNTP:ddGTP (1 mM each dNTP, 3.3 uM ddGTP) in tube G.
- 3 ul of dNTP:ddTTP (1 mM each dNTP, 3.3 uM ddTTP) in tube T.

The dNTP:ddNTP mixes are preferably heated to 85°C when added to the tube. The reaction mixture is mixed well and it is subjected to the following thermal cycling regime for 55 cycles:

94°C/30 sec.

60°C/30 sec.
70°C/1 min

After the last cycle, the tubes are kept at 70°C for 2 min, then cooled to 4°C until ready for loading. To view the reaction products, 6 ul of loading buffer (dye/stop solution) is added to each tube. The aqueous phase (the bottom phase disposed under the oil layer) is removed and put it in another tube. The sample is heated to 75°C for 3 min, and put on ice. 2 ul of each sample is loaded in each well of a MicroGene Blaster automated DNA sequencer (Visible Genetics Inc., Toronto, ON). The reaction products are electrophoretically separated and detected. The data is analyzed using GeneObjects software (Visible Genetics Inc., Toronto, ON) to base-call (i.e. determine the DNA sequence) of the samples. The base-called sequence is compared to the known *C. trachomatis* sequence to confirm diagnosis. Results are reported to the patient file.

EXAMPLE 8

The method of the invention may be employed to identify not only the presence of *C. trachomatis* in a patient sample but also the strain identity. Health care workers currently seek to distinguish among *Chlamydia trachomatis* strains to determine the molecular epidemiologic association of a range of diseases with infecting genotype (See Dean, D. et al "Major Outer Membrane Protein Variants of *Chlamydia trachomatis* Are Associated with Severe Upper Genital Tract Infections and Histopathology in San Francisco." J. Infect. Dis. 172:1013-22 (1995)).

A suitable strain specific target for C. trachomatis is the ompl (outer membrane protein) gene which has at least 4 variable sequence ("VS") domains that may be used to distinguish among the 15 known genotypes of C. trachomatis (Yuan, Y et al. "Nucleotide and Deduced Amino Acid Sequences for the Four Variable Domains of the Major Outer Membrane Proteins of the 15 *Chlamydia trachomatis* Serovars" Infect. Immun. 57 1040-1049 (1989)).

Strain identification is achieved using the method of Example 1 with the following modifications. First of all, because of the length of the VS domains, separate reactions are performed to obtain sequence from VS1/VS2 and VS3/VS4. The following oligonucleotide primers may be employed:

For VS1/VS2:

| Name | Sequence | |
|---|---|---|
| MF21 | CCGACCGCGT CTTGAAAACA GATGT | [SEQ. ID NO. 40] |
| MB22 | CACCCACATT CCCAGAGAGC T | [SEQ. ID NO. 41] |

For VS3/VS4

| Name | Sequence | |
|---|---|---|
| MVF3 | CGTGCAGCTT TGTGGGAATG T | [SEQ. ID NO. 42] |
| MB4 | CTAGATTTCA TCTTGTTCAA TTGC | [SEQ. ID NO. 43] |

These sequencing primers were employed previously for PCR amplification reactions, but not sequencing. Mahoney et al., *supra.*

These oligonucleotide primers are used in separate reactions in place of KL1 and KL2 in Example 1. The sample preparation and sequencing reactions are performed as in Example 1. The reaction products are electrophoretically separated and detected on a MicroGene Blaster automated DNA sequencing apparatus (Visible Genetics Inc., Toronto, ON). The data is analyzed using GeneObjects software to base-call the samples and to compare the data to the known varieties of *C. trachomatis.* Pure populations generally give unambiguous sequence data. Where heterozygous mixed populations are detected, a circumstance thought to occur in 1-3% of clinical *C. trachomatis* samples, the software identifies the strains which could be combined to result in the particular heterozygote sample detected.

EXAMPLE 9

Strain-specific *C. trachomatis* identification over the VS1/VS2 domain can be achieved according to the method in Example 7, by using the following degenerate primers sets:

**Forward**

**OMP291: AGCATGCGTR TKGGTTACTA YGG [SEQ ID NO. 44]**

(labeled with Cy5.5). Base 175 to 197 of the ORF of the omp1 gene of *C. trachomatis.*

**Forward**

**OMP314A: TGACTTTGTT TTCGACCGYG TTTT [SEQ ID NO. 45]**

(labeled with Cy5.5). Base 198 to 221 of the ORF of the omp1 gene of C. trachomatis.

**Reverse**

**OMP722: CTAAAGTYGC RCATCCACAT TCC [SEQ ID NO. 46]**

Base 637 to 615 of the ORF of the omp1 (in serovar K) gene of C. trachomatis. The primer may not have the exact same sequence as in serovar K.

**Reverse**

**OMP711: CATCCACATT CCCASARAGC TGC [SEQ ID NO. 47]**

Base 626 to 604 of the ORF of the omp1 (in serovar K) gene of C. trachomatis. The primer may not have the exact same sequence as in serovar K.

These primers sets are preferably used in the following combinations:

(1) OMP291-OMP722, sequencing a 455 to 463-bp (depending on the serotype) fragment of the ompl gene of C. *trachomatis*; or
(2) OMP314A-OMP711, sequencing a 421 to 430-bp (depending on the serotype) fragment of the omp1 gene of *C. trachomatis.*

EXAMPLE 10

The method as exemplified in Examples 7, 8 and 9 may be further improved by employing different labels, preferably fluorescent labels, on the different primers for use in a multi-dye sequencer. This method takes advantage of the fact that a given termination mixture containing, for example, ddATP will give chain termination products for the A nucleotide in both directions. The different primer labels means that one reaction mixture loaded in a single lane of an automated DNA sequencing apparatus designed to detect the two labels (a "multi-dye sequencer") will identify the A nucleotide of both sense and antisense strands. Separate reactions are performed for the other 3 nucleotides. Using only 4 lanes of an electrophoresis gel. and 4 reaction mixtures, the DNA sequences of both the sense and anti-sense strands can be obtained. This information allows the operator to resolve any ambiguities that may be present.

Use of two different labels lends itself to a further improvement. As noted above, in a reaction according to the invention, the results of the ddATP reaction will give chain termination products for the A nucleotide in both directions. Since the A nucleotide in one direction corresponds to the T nucleotide in the other, a single reaction can provide the location of two bases. A second termination reaction with, for example, ddCTP will then obtain the positions of the other two nucleotides, C and G. Thus only two lanes of an electrophoresis gel and 2 reaction mixtures are required to identify the location of all 4 bases of the sequence.

A suitable multi-dye sequencer for use with this aspect of the invention, is the Applied Biosystems 377 Prism automated DNA sequencer (Applied Biosystems Inc., Foster City, CA). The fluorescent labels are selected to be detectable on the 377 instrument. Instead of the dye-terminator chemistry suggested in the Applied Biosystems product literature, however, the fluorescent labels must be conjugated to the 5' end of the primer molecules. The samples are electrophoresed, detected and the detected data is recorded.

Sophisticated software such as GeneObjects software (Visible Genetics Inc, Toronto, CA) may be used to assist in evaluation of the results. This software may employ the methods of commonly assigned US Patent Applications Nos. 08/497,202 and 08/670,534 and International Patent Application No. PCT/US96/11130, all of which are incorporated herein by reference. In one of the methods, the single nucleotide data tracks are evaluated and nucleotides are positioned relative to the known (or standard) DNA sequence expected from the sample. When data tracks are generated for each of the four nucleotides, the full DNA sequence of the sample may be base-called. The base-called sequence is then compared to the library of known sequences to determine which C. trachomatis strain or strains are present in the sample.

EXAMPLE 11

The sequence of both the sense strand and antisense strand of a *C. trachomatis* cryptic plasmid gene may be obtained in a one step reaction using the primers:

| Name | Sequence | |
|---|---|---|
| KL1: | TCCGGAGCGA GTTACGAAGA | [SEQ ID NO. 38] |
| CT1590: | ATGCCCGGGA TTGGTTGATC | [SEQ ID NO. 48] |

Combine the following materials and mix well:

| | Concentration | Amount |
|---|---|---|
| Patient Sample DNA | | 11.25 ul |
| KL1*Cy5.5 Primer | 10 uM | 3 ul |
| CT1590*Fluorescein Primer | 10 uM | 2 ul |
| Enzyme Diluent (Amersham plc) | | 8 ul |
| Thermo Sequenase Enzyme | 32 U/ul | 0.9 ul |
| double distilled $H_2O$ | | 24.2 ul |

Take 11 ul of the mixture and add 2 ul of 13X buffer [Tris-HCl 260 mM pH 8.3, $MgCl_2$ 39 mM] (final concentration 20 mM Tris-HCl pH 8.3, 3 mM $MgCl_2$). Mix well and place 3 ul into each of 4 tubes. Heat tube to 94°C for 5 mins then reduce temperature to 85°C. Add and mix 3 ul of an 85 C dNTP/ddNTP solution consisting of 0.75 mM each dNTP and 2.5 uM of a chain terminating nucleotide triphosphate (ddNTP) (use a different ddNTP in each of the 4 tubes).

Treat the mixture to 60 cycles of the following thermal cycling reactions: 94°C for 10 sec, 62°C for 15 sec, 70°C for 1 min. Upon completion, treat the mixture for a final 5 min at 70 C and then store at 4°C until ready for loading. For viewing the reaction products, add an equal volume of stop/loading solution (95% formamide plus a colored dye). Take 1.5 ul and load in a single lane of a MicroGene Blaster automated DNA sequencer (Visible Genetics Inc., Toronto). Load the remaining mixture (@ 10.5 ul) in a single lane of an ALF Automated Sequencer (Pharrnacia LKB, Uppsala, Sweden). The reaction products from the Cy5.5 labeled primer are detected on the MicroGene Blaster using GeneObjects Software. The reaction products from the fluorescein labeled primer are detected on the ALF Automated Sequencer using GeneObjects Software. The base-calling results of the Cy5.5 labeled primer were compared to the known sequence of the gene by the GeneLibrarian component of GeneObjects.

EXAMPLE 12

As described in U.S. Patent Application Serial No. 08/577,858, not all 4 nucleotides of *C. trachomatis*, or any polymorphic or multiple allelic locus of any gene or organism necessarily need to be determined in order to ascertain which allele or variant is present. In many cases, positioning less than four nucleotides may be sufficient to determine with certainty which allele is present. The method of Examples 7-10 may be modified to obtain single nucleotide data tracks (or fragment patterns) by performing only one of the termination reactions at a time.

In the case of detection and serotyping of *C. trachomatis,* the evaluation of the A track alone over the first 100 nucleotides of the *omp1* gene, aligning to nucleotides 249-349 of the serovars C and K, can distinguish the serovars. Appendix I is a text file representation of the *omp1* gene in each of the serovars. The sequences are all aligned to the last (3') nucleotide of the detectably labeled primer omp314A. (Appendix I shows sequences starting 29 bp downstream of the 3'-nucleotide.) This illustration differs from a traditional "consensus" sequence illustrations in that all missing bases (usually represented by N's or raised dashes) are deleted. The A's are illustrated in the order and positions in which they would be expected to appear after a sequencing reaction and upon detection by an automated DNA electrophoresis apparatus.

If, in another microorganism, the A lane (or other preferred first lane) were not sufficient to distinguish all types, a second reaction for the C, G or T nucleotide could be performed to further define the qualitative nature of any target microorganism present in the sample. Because the sequences of the types are previously known, the operator can determine which of the nucleotides provide the greatest information and will analyze those nucleotides first.

<u>EXAMPLE 13</u>

The presence of and strain identity of *C. trachomatis* in a patient sample may be determined according to the methods of the previous examples by substituting the following primer pairs. These primers are used to determine the sequence of the ompl gene (publicly available at GENBANK Accession No. X62921).
Forward Primer (5' Primer) labeled with a detectable label such as Cy5.5:

**Primer OMP312: GGAGACTTTG TTTTCGACCG [SEQ ID NO 49]**
**Position 312-331 of X62921**

and one of the following Reverse Primers (3' Primer) (optionally labeled with a detectable label different from the 5' primer):

**Primer OMP708: CATTCCCACA AAGCTGCGCG [SEQ ID NO 50]**
**Position 727-708 of X62921**

**Primer OMP706: TTCCCACAAA GCTGCGCGAG [SEQ ID NO 51]**
**Position 725-706 of X62921**

**Primer OMP704: CCCACAAAGC TGCGCGAGCG [SEQ ID NO 52]**
**Position 723-704 of X62921**

The following combination can be used to obtain DNA sequence over the following maximum lengths:

OMP312-OMP708: 416-nt region of omp1
OMP312-OMP706: 414-nt region of omp1
OMP312-OMP704: 412-nt region of omp1

<u>EXAMPLE 14</u>

The presence of and strain identity of C. trachomatis in a patient sample may be determined according to the method of previous examples, using C, trachomatis ribosomal DNA (rDNA) specific primers such as

**CT220 ACCTTTCGGT TGAGGGAGAG TCTA [SEQ ID NO 53]**

and

CT447 GGACCAATTC TTATTCCCAA GCGA [SEQ ID NO 54]

Haydock et al., Chap 1.10 in Persing et al., *supra.*

EXAMPLE 15

The sequence of both the sense strand and antisense strand of the protease gene of HIV-1 integrated into natural abundance DNA of lymphocytes may be obtained in a one step reaction as follows.

Natural abundance DNA is prepared from the patient blood lymphocyte sample according to a standard method such as a standard salting-out procedure (as provided by the Puregene DNA Isolation Kit, Gentra Systems, Inc., Minneapolis) or by detergent and proteinase K treatment (Current Protocols in Molecular Biology, Eds. Ausubel, F.M. et al, (John Wiley & Sons; 1995)).

Combine the following materials and mix well:

| | Concentration | Amount |
|---|---|---|
| Patient Sample DNA | | 11.25 ul |
| PR211F*Cy5.5 Primer | 10 uM | 3 ul |
| or | | |
| PR281*Cy5.5 Primer | 10 uM | 3 ul |
| PR526*Fluorescein Primer | 10 uM | 2 ul |
| Enzyme Diluent (Amersham | plc) | 8 ul |
| THERMO SEQUENASE Enzyme | 32 U/ul | 0.9 ul |
| double distilled H2O | | 24.2 ul |

The primers have the following sequences:

Name Sequence

Choice of Forward Primers

PR211F ATCACTCTTT GGCAACGACC [SEQ ID No. 55]

(FORWARD), BASE 6 TO 25 OF THE PROTEASE GENE

PR281 CAGGAGCAGA TGATACAGTA TTAG [SEQ ID No. 56]

(FORWARD), BASE 76 TO 99 OF THE PROTEASE GENE

Reverse Primer

PR526: CCATTCCTGG CTTTAATTTT ACTGG [SEQ ID No. 57]

(REVERSE), BASES 321 TO 345 OF THE PROTEASE GENE

PR211F-PR526 creates a sequencing fragment of maximum size 340 bp. PR281-PR526 creates a sequencing fragment of maximum size 270 bp. Both regions contain the sequence of the various codons where mutations are involved in protease inhibitor resistance (Codons 46, 48, 54, 63 82 84 and 90).

Take 11 ul of the mixture and add 2 ul of 13X buffer [Tris-HCl 260 mM pH 8.3, $MgCl_2$ 39 mM] (final concentration 20 mM Tris-HCl pH 8.3, 3 mM $MgCl_2$). Mix well and place 3 ul into each of 4 tubes. Heat tube to 94 C for 5 mins then reduce temperature to 85 C. Add and mix 3 ul of an 85 C dNTP/ddNTP solution consisting of 0.75 mM each dNTP and 2.5 uM of a chain terminating nucleotide triphosphate (ddNTP) (use a different ddNTP in each of the 4 tubes).

Treat the mixture to 60 cycles of the following thermal cycling reactions: 94 C for 10 sec, 62 C for 15 sec, 70 C for 1 min. Upon completion, treat the mixture for a final 5 min at 70 C and then store at 4 C until ready for loading. For viewing the reaction products, add an equal volume of stop/loading solution (95% formamide plus a colored dye). Take 1.5 ul and load in a single lane of a MicroGene Blaster automated DNA sequencer (Visible Genetics Inc., Toronto). Load the remaining mixture (@ 10.5 ul) in a single lane of an ALF Automated Sequencer (Pharmacia LKB, Uppsala, Sweden). The reaction products from the Cy5.5 labeled primer are detected on the MicroGene Blaster using Gene-Objects Software. The reaction products from the fluorescein labeled primer are detected on the ALF Automated Sequencer using GeneObjects Software. The base-called results from each primer were compared to the known sequences of HIV-1 by GeneLibrarian (a component of GeneObjects (Visible Genetics Inc, Toronto).

EXAMPLE 16

The presence and type of human papilloma virus (HPV) present in a patient sample can be determined according to the method of the invention by following the protocol in Example 1 with the following modifications.

Patient sample DNA is extracted from 250 ul urine specimens using Geneclean II (Bio 101, Inc.). The sample is then treated as described previously but employing the degenerate primer pair:

Forward Primer: MY11

GCMCAGGGWC ATAAYAATGG [SEQ ID No. 58]

Reverse Primer: MY09

CGTCCMAARG GAWACTGATC [SEQ ID No. 59]

The reactions are performed as before, using Thermo Sequenase enzyme or the like. Reaction products are detected on an automated electrophoresis/detection device such as the MicroGene Blaster. The sequence is analyzed and compared to the known varieties of HPV to identify the type. The result is reported to the patient file.

## Claims

**1.** A method for determining the position of at least one selected species of nucleotide within a region of interest in a target nucleic acid polymer in a sample comprising the steps of combining the sample with a reaction mixture containing at least two oligonucleotide sequencing primers which, when hybridized to the target DNA, are oriented to allow chain extension towards cach other across the region of interest, at least one of said primers being labeled with a fluorescent label to synthesize chain-extension products indicative of the positions of the selected species of nucleotide within the region of interest and evaluating the products thus produced, **characterized in that** the sample which is combined with the reaction mixture contains target and non-target nucleic acid polymers in natural abundance, **in that** the products are evaluated by detection of a fluorescent label attached to the sequencing primer, and **in that** the reaction mixture comprises a thermally-stable polymerase enzyme which incorporates dideoxynucleotides into an extending nucleic acid polymer at a rate which is no less than 0.4 times the rate of incorporation of deoxynuclcotides.

**2.** A method according to claim 1, comprising the steps of:

(a) combining the sample with first and second primers, a nucleotide triphosphate feedstock mixture, a chain-terminating nucleotide triphosphate and a thermally stable polymerase enzyme to form a reaction mixture, said first and second primers binding to the sense and antisense strands, respectively, of the target nucleic acid polymer at locations flanking the selected region, at least one of said primers being labeled with a fluorscent label;
(b) exposing the reaction mixture to a plurality of temperature cycles each of which includes at least a high temperature denaturation phase and a lower temperature extension phase, thereby producing a plurality of terminated fragments; and
(c) evaluating terminated fragments produced during the additional cycles to determine the positions of the nucleic acid corresponding to the chain-terminating nucleotide triphosphate within the selected region, **characterized in that** the sample contains target nucleic acid polymer and non-target nucleic acid polymer in natural abundance and that the polymerase is one which incorporates dideoxynucleotides into an extending nucleic acid polymer at a rate which is no less than 0.4 times the rate of incorporation of deoxynucleotides.

**3.** The method of claim 2, wherein the mole ratio of the dideoxynucleotide triphosphate to the corresponding deoxynucleotide triphosphate in the reaction mixture is from 1:50 to 1:1000.

**4.** The method of claim 2, wherein the mole ratio of the dideoxynucleotide triphosphate to the corresponding deoxynucleotide triphosphate is from 1:100 to 1:300.

**5.** The method of any of claims 1 or 2 to 4, wherein the primers are each labeled with a different fluorescent label.

**6.** The method of any of claims 1 or 2 to 5, wherein the polymerase enzyme is Thermo Sequenase™.

**7.** The method according to any of claims 1 or 2 or 6, wherein the reaction mixture further comprises a second polymerase enzyme having a low affinity for incorporation of dideoxynucleotide triphosphates compared to deoxynucleotide triphosphates.

**8.** The method according to claim 7, wherein the second polymerase is Taq polymerase.

**9.** The method according to any of claims 1 or 2 to 8, wherein the sample is human genomic DNA.

**10.** The method according to any of claims 1 or 2 to 8, wherein the sample is a complex sample containing just nuclear DNA, or just mitochondrial DNA or some subtraction of nuclear or mitochondrial DNA obtained by isolation from a tissue sample.

**11.** The method according to any of claims 1 or 2 to 8, wherein the sample is a DNA sample prepared by conversion, for example by reverse transcription, of a total mRNA preparation or the genome of an RNA virus to cDNA.

**12.** A method for detecting a mutation in DNA in a sample, comprising determining the positions of at least one nucleotide in the DNA using the method of any of claims 1 or 2 to 11.

**13.** The method of claim 12, wherein the DNA encodes the gene for the Von Hippel-Lindau tumor suppressor.

**14.** The method of claim 12, wherein the DNA encodes the gene for the p53 tumor suppressor.

**15.** A method for determining the HLA type of a sample, comprising determining the positions of at least one nucleotide in DNA from the sample using the method of any of claims 1 or 2 to 9.

## Patentansprüche

1. Verfahren zur Bestimmung der Position mindestens einer ausgewählten Nukleotid-Spezies innerhalb einer interessierenden Region in einem Ziel-Nukleinsäure-Polymer in einer Probe, umfassend die Schritte der Vereinigung der Probe mit einer Reaktionsmischung, die mindestens zwei Oligonukleotid-Sequenzierungsprimer enthält, welche, wenn sie mit der Ziel-DNA hybridisiert sind, so orientiert sind, dass sie eine Kettenverlängerung aufeinander zu über die interessierende Region hin gestatten, wobei mindestens einer der Primer mit einer fluoreszierenden Markierung markiert ist, um Kettenverlängerungsprodukte zu synthetisieren, welche die Positionen der ausgewählten Nukleotid-Spezies innerhalb der interessierenden Region anzeigen, und der Auswertung der so erzeugten Produkte, **dadurch gekennzeichnet, dass** die Probe, die mit der Reaktionsmischung vereinigt wird, Ziel- und Nicht-Ziel-Nukleinsäure-Polymere in natürlicher Häufigkeit enthält, dass die Produkte durch Nachweis einer fluoreszierenden Markierung ausgewertet werden, welche an dem Sequenzierungsprimer angebracht ist, und dass die Reaktionsmischung ein thermisch stabiles Polymeraseenzym umfasst, welches Didesoxynukleotide in ein sich verlängerndes Nukleinsäure-Polymer mit einer Geschwindigkeit einbauen, die nicht geringer ist als das 0,4-fache der Geschwindigkeit des Einbaus von Desoxynukleotiden.

2. Verfahren nach Anspruch 1, umfassend die Schritte:

   (a) Vereinigen der Probe mit einem ersten und zweiten Primer, einer Nukleotidtriphosphat-Vorratsmischung, einem kettenabbrechenden Nukleotidtriphosphat und einem thermisch stabilen Polymeraseenzym, um eine Reaktionsmischung zu bilden, wobei sich der erste und zweite Primer an den Sinn- bzw. Antisinn-Strang des Ziel-Nukleinsäure-Polymers an Orten bindet, welche die ausgewählte Region flankieren, wobei mindestens einer der Primer mit einer fluoreszierenden Markierung markiert ist;
   (b) Einwirkenlassen einer Mehrzahl von Temperaturzyklen auf die Reaktionsmischung, von denen jeder mindestens eine Hochtemperatur-Denaturierungsphase und eine Verlängerungsphase bei niedrigerer Temperatur einschließt, wodurch eine Mehrzahl von kettenabgebrochenen Fragmenten erzeugt wird; und
   (c) Auswertung der kettenabgebrochenen Fragmente, die bei den zusätzlichen Zyklen erzeugt werden, um die Positionen der Nukleinsäure, welche dem kettenabbrechenden Nukleotidtriphosphat entspricht, innerhalb der ausgewählten Region zu bestimmen, **dadurch gekennzeichnet, dass** die Probe Ziel-Nukleinsäure-Polymer und Nicht-Ziel-Nukleinsäure-Polymer in natürlicher Häufigkeit enthält und dass die Polymerase die Didesoxynukleotide in ein sich verlängerndes Nukleinsäure-Polymer mit einer Geschwindigkeit einbaut, die nicht geringer ist als das 0,4-fache der Geschwindigkeit des Einbaus von Desoxynukleotiden.

3. Verfahren nach Anspruch 2, in dem das Molverhältnis des Didesoxynukleotidtriphosphats zu dem entsprechenden Desoxynukleotidtriphosphat in der Reaktionsmischung 1:50 bis 1:1000 beträgt.

4. Verfahren nach Anspruch 2, in dem das Molverhältnis des Didesoxynukleotidtriphosphats zu dem entsprechenden Desoxynukleotidtriphosphat 1:100 bis 1:300 beträgt.

5. Verfahren nach irgendeinem der Ansprüche 1 oder 2 bis 4, in dem jeder Primer mit einer unterschiedlichen fluoreszierenden Markierung markiert ist.

6. Verfahren nach irgendeinem der Ansprüche 1 oder 2 bis 5, in dem das Polymeraseenzym Thermo Sequenase™ ist.

7. Verfahren nach irgendeinem der Ansprüche 1 oder 2 bis 6, in dem die Reaktionsmischung weiter ein zweites Polymeraseenzym mit einer niedrigen Affinität zum Einbau von Didesoxynukleotidtriphosphaten im Vergleich zu Desoxynukleotidtriphosphaten umfasst.

8. Verfahren nach Anspruch 7, in dem die zweite Polymerase Taq-Polymerase ist.

9. Verfahren nach irgendeinem der Ansprüche 1 oder 2 bis 8, in dem die Probe menschliche genomische DNA ist.

10. Verfahren nach irgendeinem der Ansprüche 1 oder 2 bis 8, in dem die Probe eine komplexe Probe ist, die nur Zellkern-DNA oder nur Mitochondrien-DNA oder irgendeine Unterfraktion von Zellkern- oder Mitochondrien-DNA enthält, die aus einer Gewebeprobe durch Isolierung erhalten wird.

11. Verfahren nach irgendeinem der Ansprüche 1 oder 2 bis 8, in dem die Probe eine DNA-Probe ist, die durch Umwandlung, z.B. durch reverse Transkription, eines Gesamt-mRNA-Präparats oder des Genoms einer RNA-Virus in cDNA hergestellt ist.

**12.** Verfahren zum Nachweis einer Mutation in einer DNA in einer Probe, umfassend die Bestimmung der Positionen von mindestens einem Nukleotid in der DNA unter Verwendung des Verfahrens von irgendeinem der Ansprüche 1 oder 2 bis 11.

**13.** Verfahren nach Anspruch 12, in dem die DNA für das Gen des von Hippel-Lindau-Tumorsuppressors kodiert.

**14.** Verfahren nach Anspruch 12, in dem die DNA für das Gen des p53-Tumorsuppressors kodiert.

**15.** Verfahren zur Bestimmung des HLA-Typs einer Probe, umfassend das Bestimmen der Positionen mindestens eines Nukleotids in DNA aus der Probe unter Verwendung des Verfahrens nach irgendeinem der Ansprüche 1 oder 2 bis 9.

**Revendications**

**1.** Procédé de détermination de la position d'au moins une espèce de nucléotide choisie dans une région d'intérêt d'un polymère acide nucléique cible dans un échantillon, comprenant les étapes selon lesquelles on combine l'échantillon avec un mélange réactionnel contenant au moins deux amorces de séquençage oligonucléotidiques qui, lorsqu'elles sont hybridées à l'ADN cible, sont orientées de manière à permettre l'allongement des chaînes l'une vers l'autre à travers la région d'intérêt, au moins l'une desdites amorces étant marquée avec un marqueur fluorescent, pour synthétiser des produits d'allongement de chaînes indiquant les positions des espèces de nucléotides choisies dans la région d'intérêt, et on évalue les produits ainsi obtenus, **caractérisé en ce que** l'échantillon combiné avec le mélange réactionnel contient des polymères acides nucléiques cibles et non cibles en une abondance naturelle, **en ce que** l'évaluation des produits s'effectue par détection d'un marqueur fluorescent lié à l'amorce de séquençage, et **en ce que** le mélange réactionnel comprend une enzyme polymérase stable à la chaleur qui incorpore des didésoxynucléotides dans un polymère acide nucléique en phase d'allongement à une vitesse d'au moins 0,4 fois la vitesse d'incorporation de désoxynucléotides.

**2.** Procédé selon la revendication 1, comprenant les étapes selon lesquelles

(a) on combine l'échantillon avec une première et une deuxième amorce, un mélange substrat de nucléotide triphosphates, un nucléotide triphosphate terminateur de chaîne et une enzyme polymérase stable à la chaleur pour former un mélange réactionnel, lesdites première et deuxième amorces se liant respectivement aux brins sens et antisens du polymère acide nucléique cible à des positions flanquant la région choisie, au moins l'une desdites amorces étant marquée avec un marqueur fluorescent;
(b) on expose le mélange réactionnel à une pluralité de cycles de température comprenant chacun au moins une phase de dénaturation à haute température et une phase d'allongement à une température plus basse, en produisant ainsi une pluralité de fragments terminés; et
(c) on évalue les fragments terminés produits pendant les cycles supplémentaires pour déterminer les positions de l'acide nucléique correspondant au nucléotide triphosphate terminateur de chaîne dans la région choisie,

**caractérisé en ce que** l'échantillon contient des polymères acides nucléiques cibles et des polymères acides nucléiques non cibles en une abondance naturelle, et **en ce que** la polymérase est une polymérase qui incorpore des didésoxynucléotides dans un polymère acide nucléique en phase d'allongement à une vitesse d'au moins 0,4 fois la vitesse d'incorporation de désoxynucléotides.

**3.** Procédé selon la revendication 2, dans lequel le rapport molaire du didésoxynucléotide triphosphate au désoxynucléotide triphosphate correspondant dans le mélange réactionnel est compris entre 1:50 et 1:1 000.

**4.** Procédé selon la revendication 2, dans lequel le rapport molaire du didésoxynucléotide triphosphate au désoxynucléotide triphosphate correspondant est compris entre 1:100 et 1:300.

**5.** Procédé selon l'une quelconque des revendications 1 ou 2 à 4, dans lequel les amorces sont marquées chacune avec un marqueur fluorescent différent.

6. Procédé selon l'une quelconque des revendications 1 ou 2 à 5, dans lequel l'enzyme polymérase est la Thermo Sequenase®.

7. Procédé selon l'une quelconque des revendications 1 ou 2 à 6, dans lequel le mélange réactionnel comprend en outre une seconde enzyme polymérase ayant une faible affinité pour l'incorporation de didésoxynucléotide triphosphates par comparaison aux désoxynucléotide triphosphates.

8. Procédé selon la revendication 7, dans lequel la seconde polymérase est la polymérase de Taq.

9. Procédé selon l'une quelconque des revendications 1 ou 2 à 8, dans lequel l'échantillon est de l'ADN génomique humain.

10. Procédé selon l'une quelconque des revendications 1 ou 2 à 8, dans lequel l'échantillon est un échantillon complexe contenant juste de l'ADN nucléaire, ou juste de l'ADN mitochondrial, ou une sous-fraction d'ADN nucléaire ou mitochondrial obtenu par isolement à partir d'un échantillon de tissu.

11. Procédé selon l'une quelconque des revendications 1 ou 2 à 8, dans lequel l'échantillon est un échantillon d'ADN préparé par conversion en ADNc, par exemple par transcription inverse, d'une préparation d'ARNm total ou du génome d'un virus à ARN.

12. Procédé de détection d'une mutation dans l'ADN d'un échantillon, comprenant la détermination des positions d'au moins un nucléotide dans l'ADN à l'aide du procédé selon l'une quelconque des revendications 1 ou 2 à 11.

13. Procédé selon la revendication 12, dans lequel l'ADN code pour le gène du suppresseur tumoral de Von Hippel-Lindau.

14. Procédé selon la revendication 12, dans lequel l'ADN code pour le gène du suppresseur tumoral p53.

15. Procédé de détermination du type HLA d'un échantillon, comprenant la détermination des positions d'au moins un nucléotide dans l'ADN de l'échantillon à l'aide du procédé selon l'une quelconque des revendications 1 ou 2 à 9.

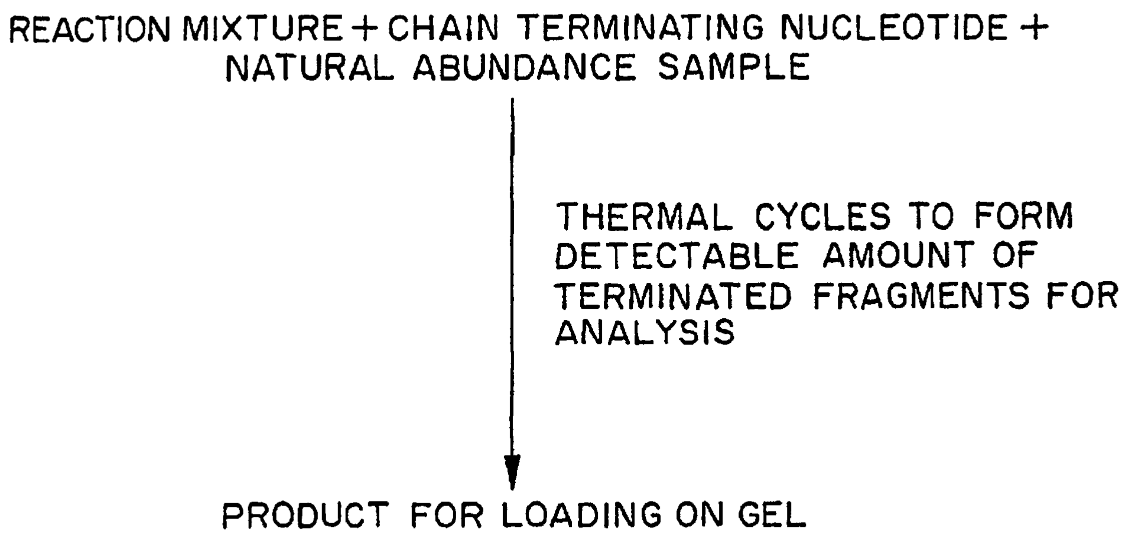
REACTION MIXTURE + CHAIN TERMINATING NUCLEOTIDE +
NATURAL ABUNDANCE SAMPLE
THERMAL CYCLES TO FORM DETECTABLE AMOUNT OF TERMINATED FRAGMENTS FOR ANALYSIS
PRODUCT FOR LOADING ON GEL

FIG. 1

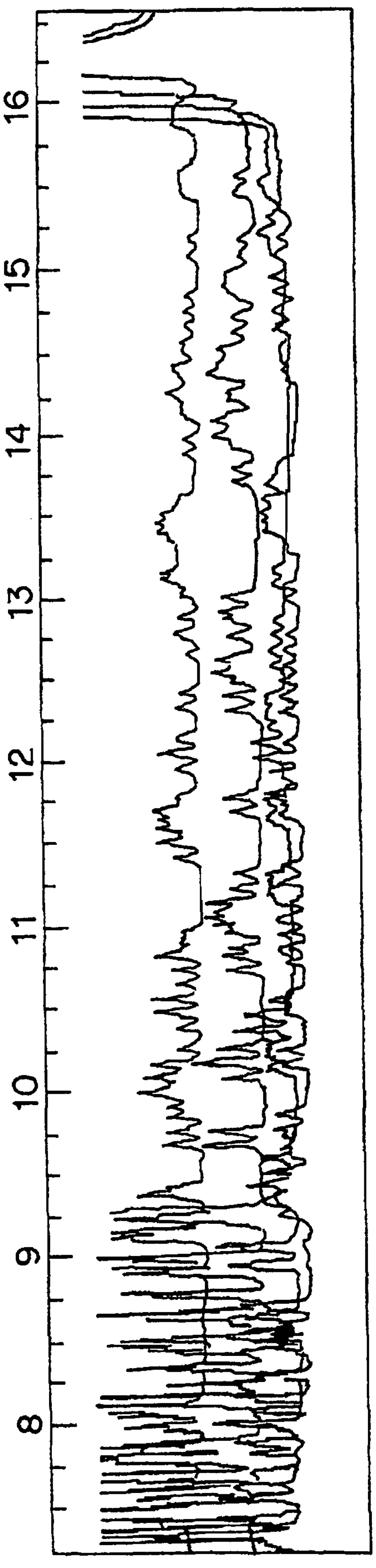


FIG. 2A

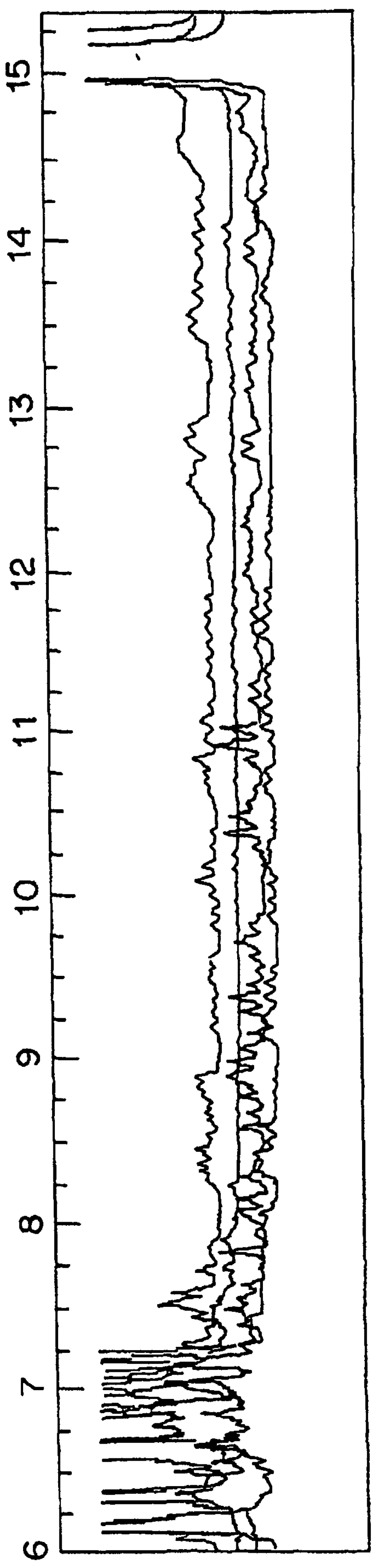


FIG. 2B

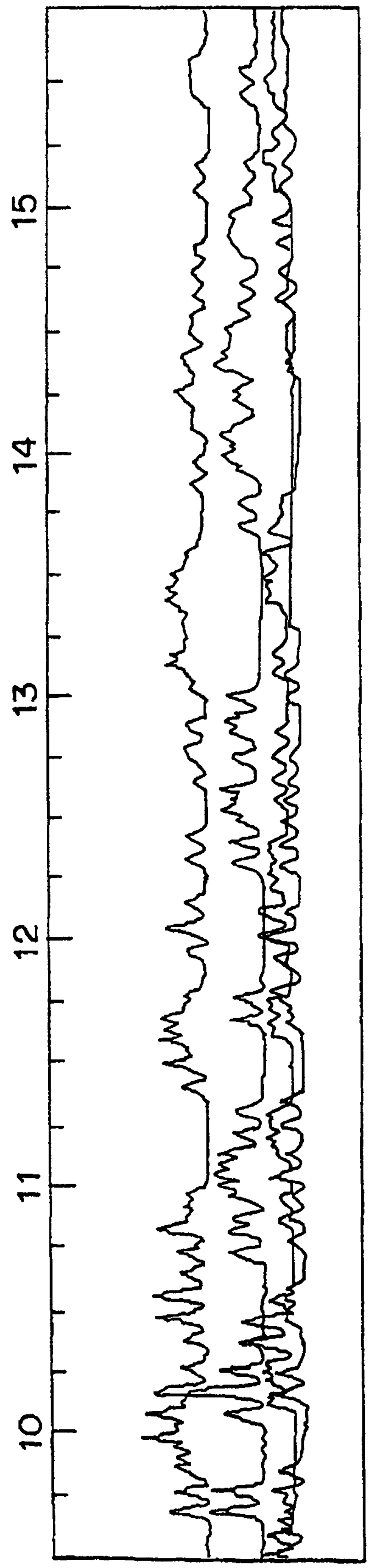


FIG. 3

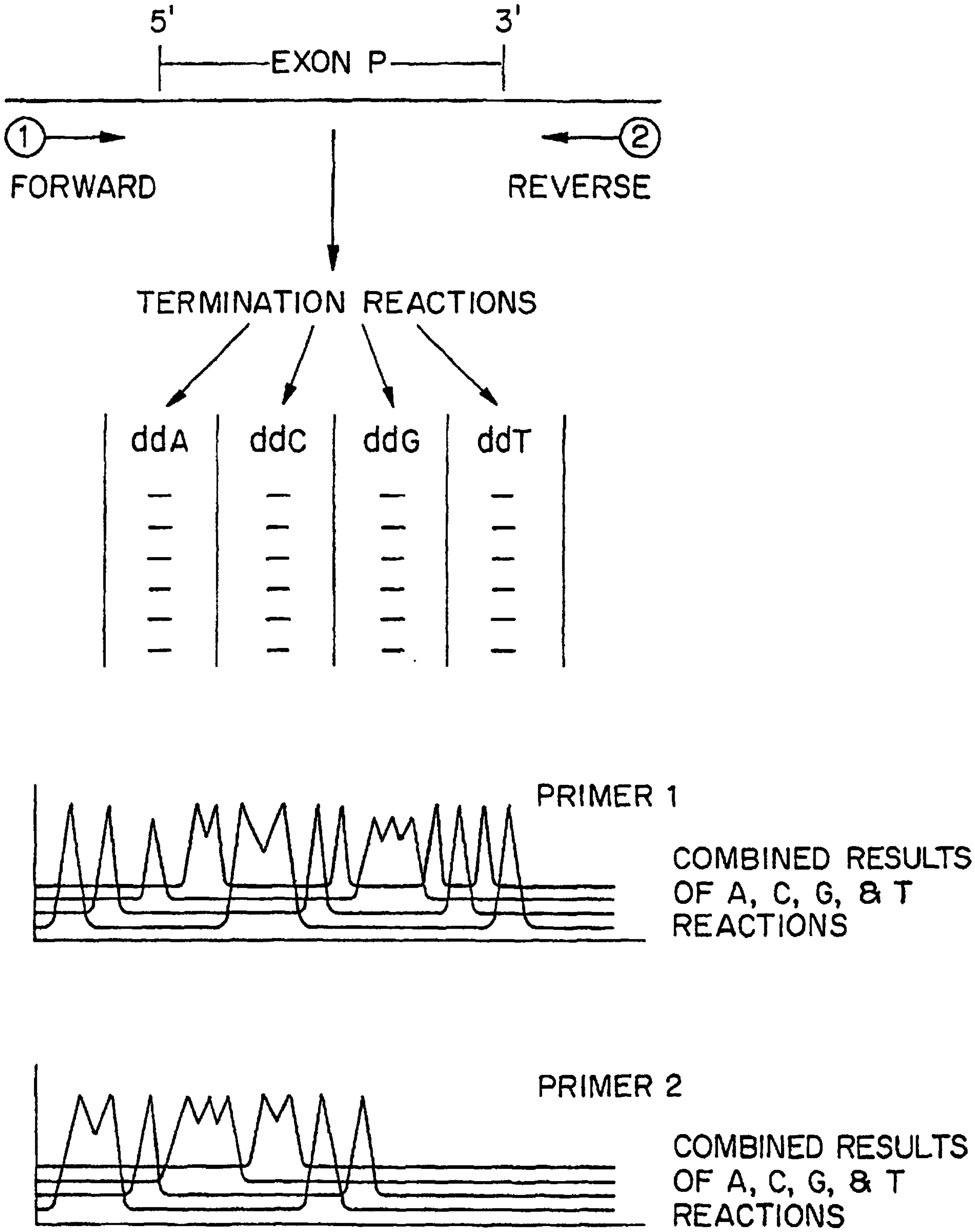
5'
3'
EXON P
1
FORWARD
2
REVERSE
TERMINATION REACTIONS
ddA
ddC
ddG
ddT
PRIMER 1
COMBINED RESULTS OF A, C, G, & T REACTIONS
PRIMER 2
COMBINED RESULTS OF A, C, G, & T REACTIONS

FIG. 4

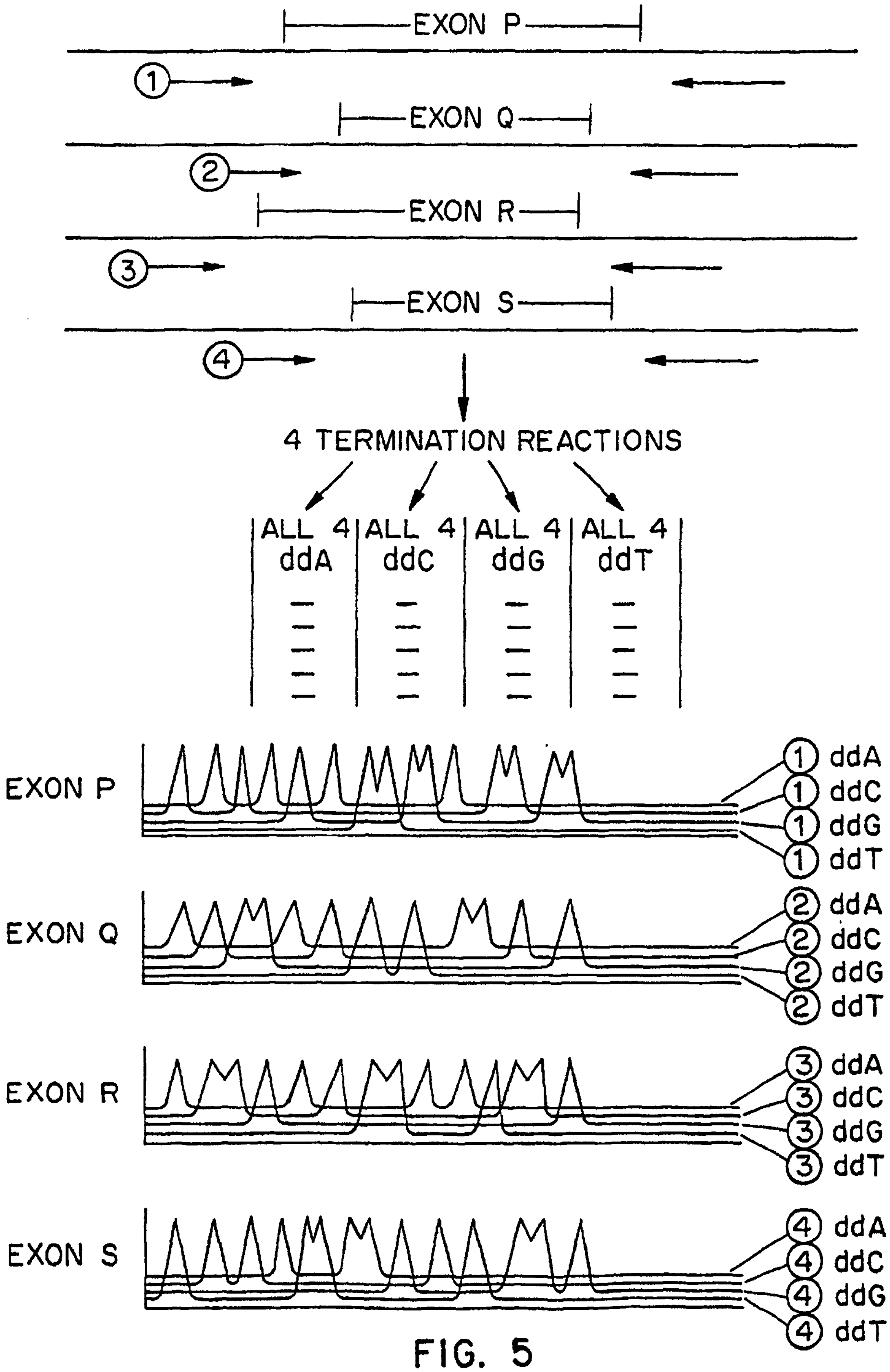
EXON P
1
EXON Q
2
EXON R
3
EXON S
4
4 TERMINATION REACTIONS
ALL 4 ddA
ALL 4 ddC
ALL 4 ddG
ALL 4 ddT
EXON P
1 ddA
1 ddC
1 ddG
1 ddT
EXON Q
2 ddA
2 ddC
2 ddG
2 ddT
EXON R
3 ddA
3 ddC
3 ddG
3 ddT
EXON S
4 ddA
4 ddC
4 ddG
4 ddT

FIG. 5

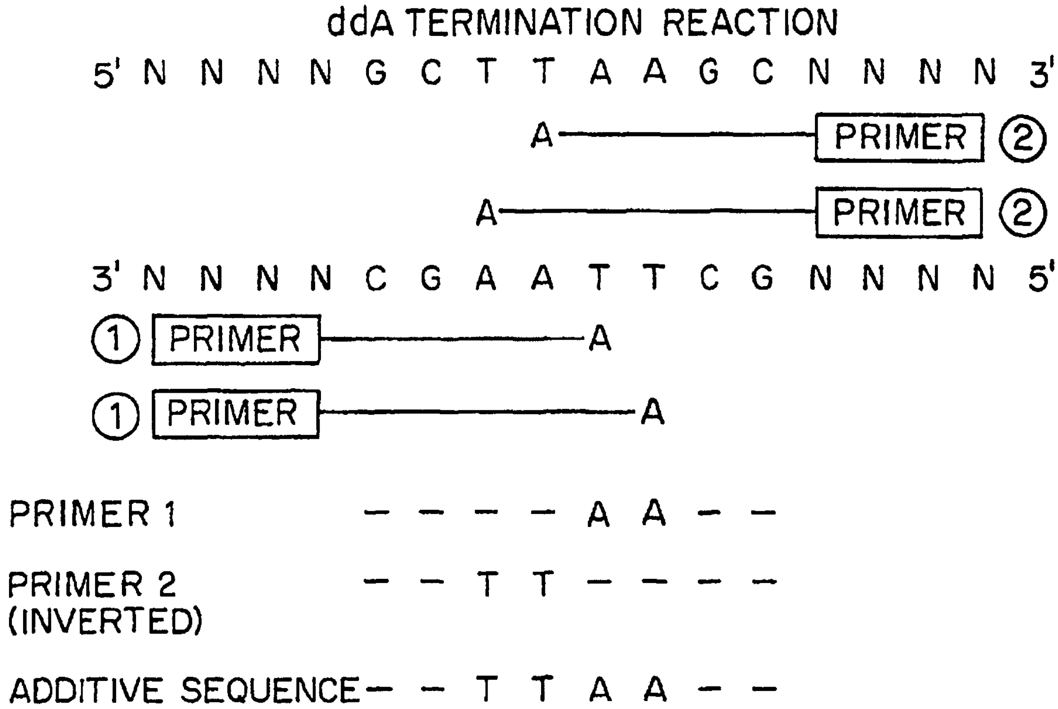
ddA TERMINATION REACTION
5' N N N N G C T T A A G C N N N N 3'
A PRIMER 2
A PRIMER 2
3' N N N N C G A A T T C G N N N N 5'
1 PRIMER A
1 PRIMER A
PRIMER 1 – – – – A A – –
PRIMER 2 (INVERTED) – – T T – – – –
ADDITIVE SEQUENCE – – T T A A – –

FIG. 6A

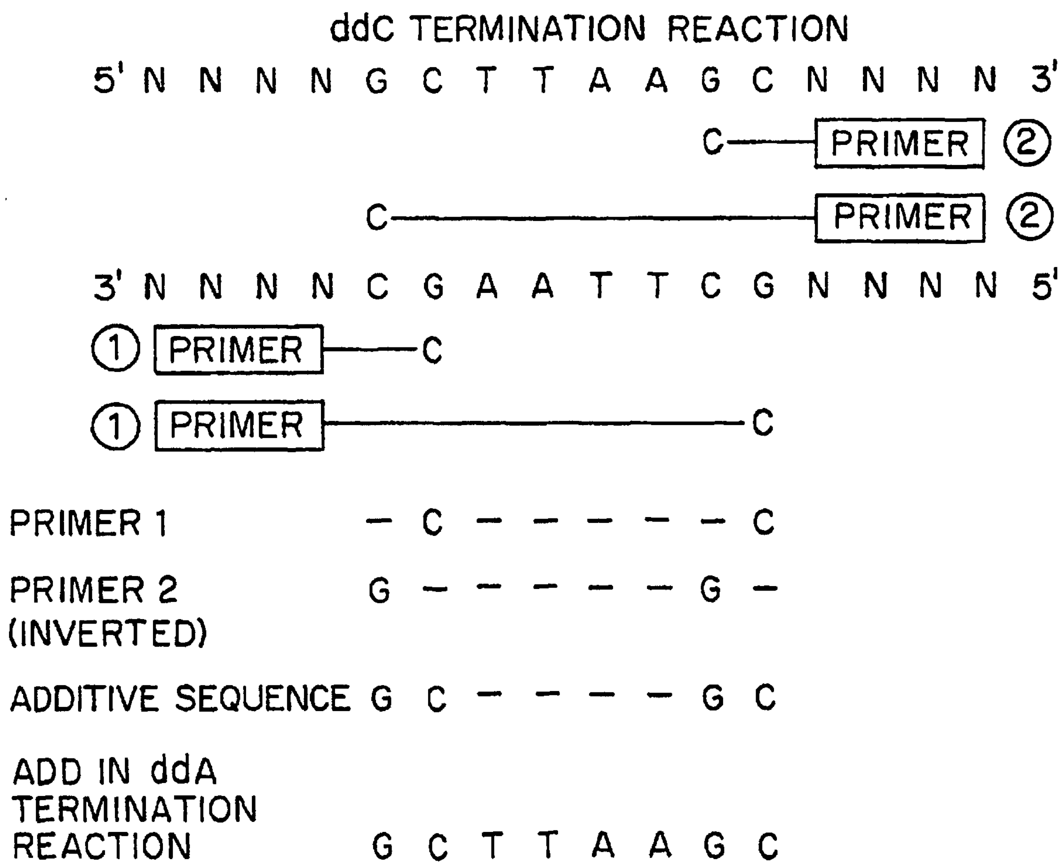
ddC TERMINATION REACTION
5' N N N N G C T T A A G C N N N N 3'
C PRIMER 2
C PRIMER 2
3' N N N N C G A A T T C G N N N N 5'
1 PRIMER C
1 PRIMER C
PRIMER 1 – C – – – – – C
PRIMER 2 (INVERTED) G – – – – – G –
ADDITIVE SEQUENCE G C – – – – G C
ADD IN ddA TERMINATION REACTION G C T T A A G C

FIG. 6B

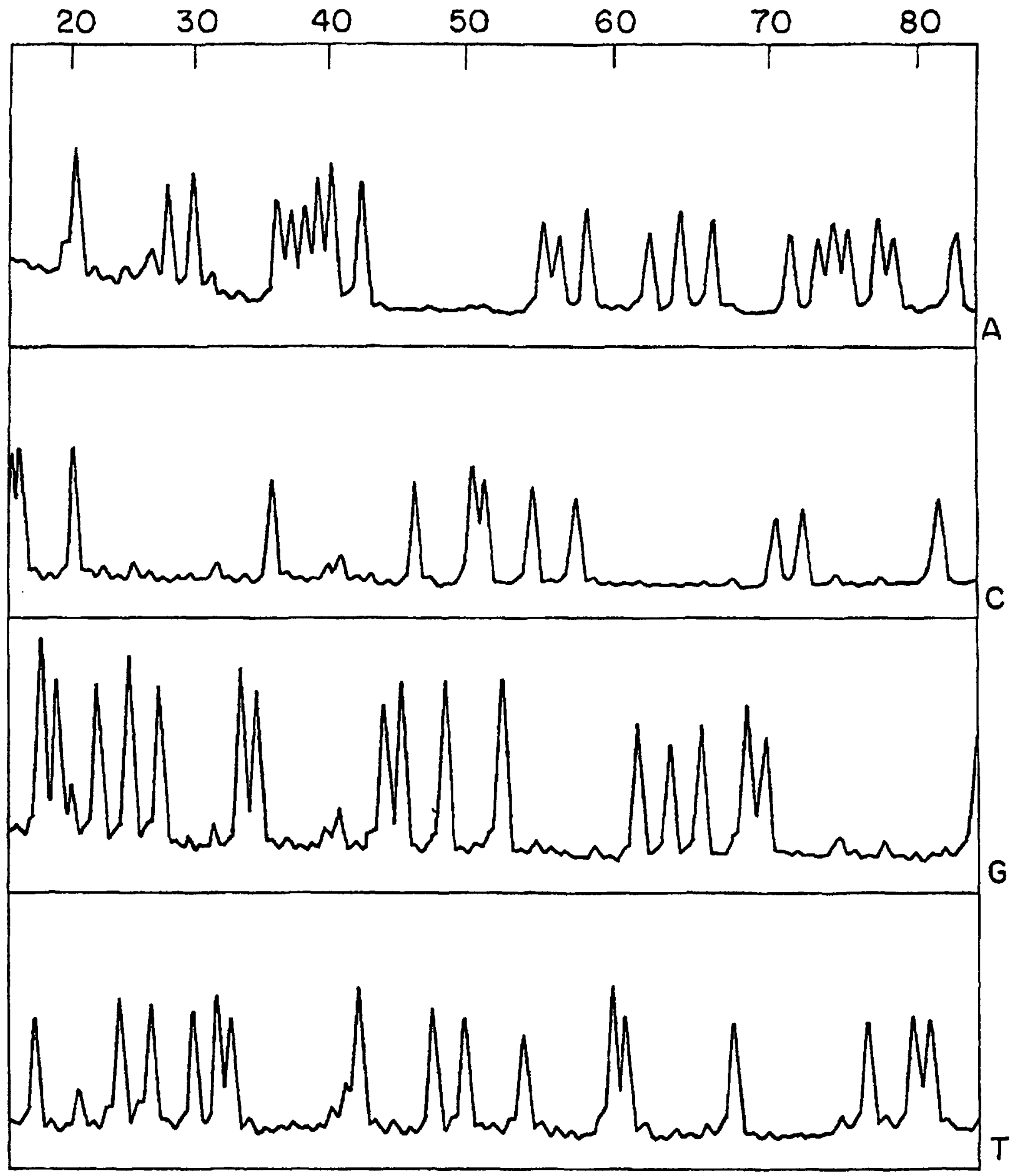


FIG. 7A

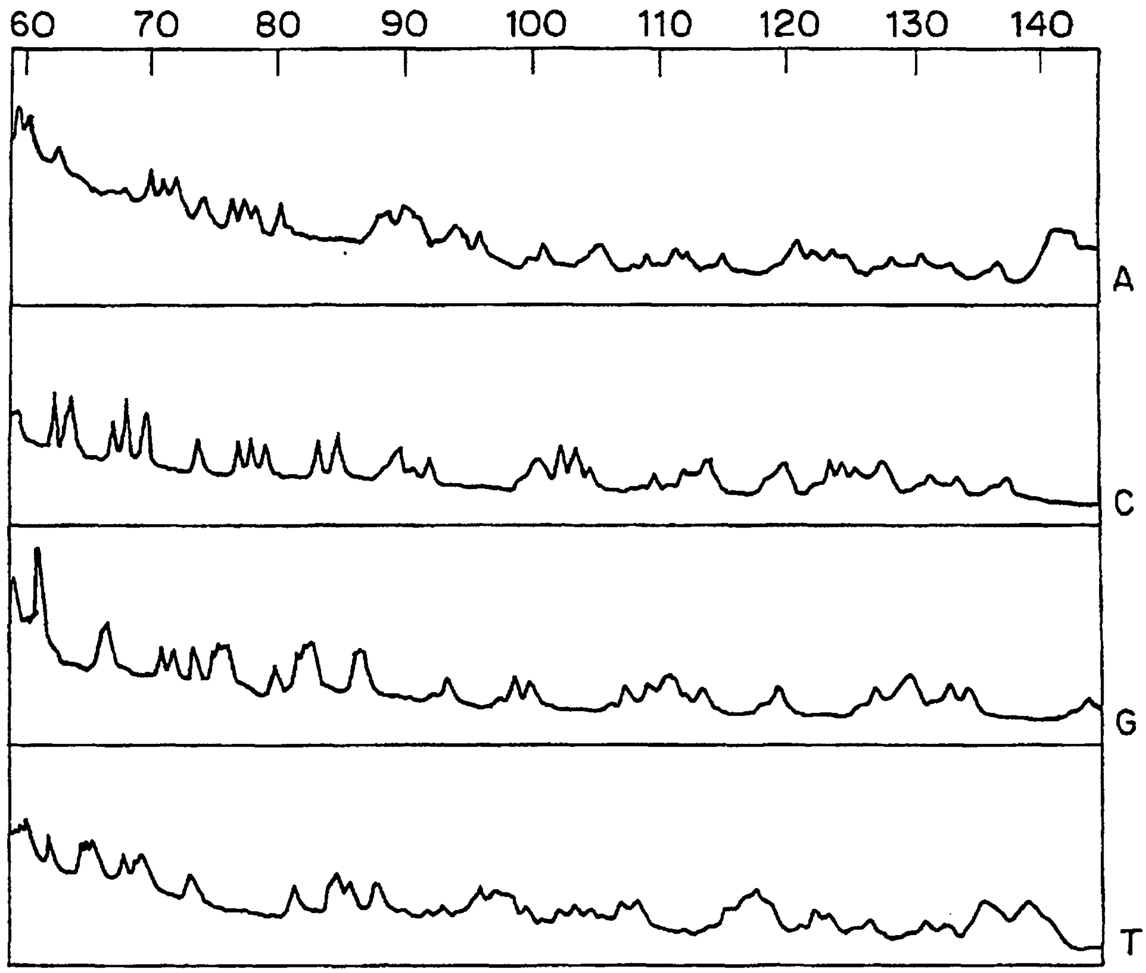


FIG. 7B

SEQUENCE LISTING

(1) GENERAL INFORMATION:

(I) APPLICANT: Visible Genetics Inc. Leushner, James
Hui, May
Dunn, James M.
Larson, Marina T.
Lacroix, Jean-Michel
Shipman, Robert
(ii) TITLE OF INVENTION: METHOD FOR SEQUENCING OF NUCLEIC ACID POLYMERS
(iii) NUMBER OF SEQUENCES: 59
(iv) CORRESPONDENCE ADDRESS:

(A) ADDRESSEE: Oppedahl & Larson
(B) STREET: 1992 Commerce Street Suite 309
(C) CITY: Yorktown
(D) STATE: NY
(E) COUNTRY: US
(F) ZIP: 10598

(v) COMPUTER READABLE FORM:

(A) MEDIUM TYPE: Diskette - 3.5 inch, 1.44 Mb storage
(B) COMPUTER: IBM compatible
(C) OPERATING SYSTEM: MS DOS
(D) SOFTWARE: Word Perfect

(vi) CURRENT APPLICATION DATA :

(A) APPLICATION NUMBER:
(B) FILING DATE:
(C) CLASSIFICATION:

(vii) PRIOR APPLICATION DATA:

(A) APPLICATION NUMBER:
(B) FILING DATE:

(viii) ATTORNEY/AGENT INFORMATION :

(A) NAME: Larson, Marina T.
(B) REGISTRATION NUMBER: 32,038
(C) REFERENCE/DOCKET NUMBER: VGEN.P-031-WO

(ix) TELECOMMUNICATION INFORMATION:

(A) TELEPHONE: (914) 245-3252
(B) TELEFAX: (914) 962-4330
(C) TELEX:

(2) INFORMATION FOR SEQ ID NO: 1:

(I) SEQUENCE CHARACTERISTICS:

(A) LENGTH: 18
(B) TYPE: nucleic acid
(C) STRANDEDNESS: double
(D) TOPOLOGY: linear

(ii) MOLECULE TYPE: other nucleic acid
(iii) HYPOTHETICAL: no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 2 of VHL gene

(xi) SEQUENCE DESCRIPTION: SEQ ID NO:1:

GGCTCTTTAA CAACCTTT 18

(2) INFORMATION FOR SEQ ID NO: 2:

(i) SEQUENCE CHARACTERISTICS:

(A) LENGTH: 22
(B) TYPE: nucleic acid
(C) STRANDEDNESS: double

(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 2 of VHL gene

(xi) SEQUENCE DESCRIPTION: SEQ ID NO:2:

GGGCTTAATT TTTCAAGTGG TC 22

(2) INFORMATION FOR SEQ ID NO: 3:

(i) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 1 of p53 gene

(xi) SEQUENCE DESCRIPTION: SEQ ID NO:3:

CGGATTACTT GCCCTTACTT GTCA 24

(2) INFORMATION FOR SEQ ID NO: 4:

(i) SEQUENCE CHARACTERISTICS:

(A) LENGTH: 19
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 1 of p53 gene

(xi) SEQUENCE DESCRIPTION: SEQ ID NO:4:

CCCCAGCCCC AGCGATTTT 19

(2) INFORMATION FOR SEQ ID NO: 5:

(i) SEQUENCE CHARACTERISTICS:

(A) LENGTH: 19
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 2 of p53 gene

(xi) SEQUENCE DESCRIPTION: SEQ ID NO:5:

CCAGGGTTGG AAGCGTCTC 19

(2) INFORMATION FOR SEQ ID NO: 6:

(i) SEQUENCE CHARACTERISTICS:

(A) LENGTH: 22
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 2 of p53 gene

(xi) SEQUENCE DESCRIPTION: SEQ ID NO:6:

GACAAGAGCA GAAAGTCAGT CC 22

(2) INFORMATION FOR SEQ ID NO: 7:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 3 of p53 gene

(xi) SEQUENCE DESCRIPTION: SEQ ID NO:7:

CATGGGACTG ACTTTCTGCT 20

(2) INFORMATION FOR SEQ ID NO: 8:

(i) SEQUENCE CHARACTERISTICS:

(A) LENGTH:18
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 3 of p53 gene

(xi) SEQUENCE DESCRIPTION: SEQ ID NO:8:

ATGGGTGAAA AGAGCAGT 18

(2) INFORMATION FOR SEQ ID NO: 9:

(i) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 4 of p53 gene

(xi) SEQUENCE DESCRIPTION: SEQ ID NO:9:

CTGGTCCTCT GACTGCTCTT TTCA 24

(2) INFORMATION FOR SEQ ID NO: 10:

(i) SEQUENCE CHARACTERISTICS:

(A) LENGTH: 22
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 4 of p53 gene

(xi) SEQUENCE DESCRIPTION: SEQ ID NO:10:

AAAGAAATGC AGGGGGATAC GG 22

(2) INFORMATION FOR SEQ ID NO: 11:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 5 of p53 gene

(xi) SEQUENCE DESCRIPTION: SEQ ID NO:11:

TGTTCACTTG TGCCCTGACT 20

(2) INFORMATION FOR SEQ ID NO: 12:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no

(v) FRAGMENT TYPE: internal
(vi) ORIGINAL SOURCE:

(A) ORGANISM: human
(D) OTHER INFORMATION: primer for sequencing of exon 5 of p53 gene

(xi) SEQUENCE DESCRIPTION: SEQ ID NO:12:

CAGCCCTGTC GTCTCTCCAG 20

(2) INFORMATION FOR SEQ ID NO: 13:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 6 of p53 gene

(xi) SEOUENCE DESCRIPTION: SEO ID NO:13:

CTGGGGCTGG AGAGACGACA 20

(2) INFORMATION FOR SEQ ID NO: 14:

(i) SEQUENCE CHARACTERISTICS:

(A) LENGTH: 19
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 6 of p53 gene

(xi) SEQUENCE DESCRIPTION: SEQ ID NO:14:

GGAGGGCCAC TGACAACCA 19

(2) INFORMATION FOR SEQ ID NO: 15:

(i) SEQUENCE CHARACTERISTICS:

(A) LENGTH: 17
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 7 of p53 gene

(xi) SEQUENCE DESCRIPTION: SEQ ID NO:15:

CTCCCCTGCT TGCCACA 17

(2) INFORMATION FOR SEQ ID NO: 16:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 7 of p53 gene

(xi) SEQUENCE DESCRIPTION: SEQ ID NO:16:

GGGTCAGCGG CAAGCAGAGG 20

(2) INFORMATION FOR SEQ ID NO: 17:

(i) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 8 of p53 gene

(xi) SEQUENCE DESCRIPTION: SEQ ID NO:17:

GACAAGGGTG GTTGGGAGTA GATG 24

(2) INFORMATION FOR SEQ ID NO: 18:

(i) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 8 of p53 gene

(xi) SEQUENCE DESCRIPTION: SEQ ID NO:18:

GCAAGGAAAG GTGATAAAAG TGAA 24

(2) INFORMATION FOR SEQ ID NO: 19:

(i) SEQUENCE CHARACTERISTICS:

(A) LENGTH: 19
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 9 of p53 gene

(xi) SEQUENCE DESCRIPTION: SEQ ID NO:19:

GCGGTGGAGG AGACCAAGG 19

(2) INFORMATION FOR SEQ ID NO: 20:

(i) SEQUENCE CHARACTERISTICS:

(A) LENGTH: 22

(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 9 of p53 gene

(xi) SEQUENCE DESCRIPTION: SEQ ID NO: 20:

AACGGCATTT TGAGTGTTAG AC 22

(2) INFORMATION FOR SEQ ID NO: 21:

(i) SEQUENCE CHARACTERISTICS:

(A) LENGTH: 23
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 10 of p53 gene

(xi) SEQUENCE DESCRIPTION: SEQ ID NO:21:

TGATCCGTCA TAAAGTCAAA CAA 23

(2) INFORMATION FOR SEQ ID NO: 22:

(i) SEQUENCE CHARACTERISTICS:

(A) LENGTH: 21
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 10 of p53 gene

(xi) SEQUENCE DESCRIPTION: SEO ID NO:22:

GTGGAGGCAA GAATGTGGTT A 21

(2) INFORMATION FOR SEQ ID NO: 23:

(i) SEQUENCE CHARACTERISTICS:

(A) LENGTH: 21
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 11 of p53 gene

(xi) SEQUENCE DESCRIPTION: SEQ ID NO:23:

GGCACAGACC CTCTCACTCA T 21

(2) INFORMATION FOR SEQ ID NO: 24:

(i) SEQUENCE CHARACTERISTICS:

(A) LENGTH: 21
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 11 of p53 gene

(xi) SEQUENCE DESCRIPTION: SEQ ID NO: 24:

TGCTTCTGAC GCACACCTAT T 21

(2) INFORMATION FOR SEQ ID NO: 25:

(i) SEQUENCE CHARACTERISTICS:

(A) LENGTH: 17
(B) TYPE: nucleic acid
(C) STRANDEDNESS: double

(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 2 of HLA-A gene

(xi) SEQUENCE DESCRIPTION: SEQ ID NO:25:

GCGCCGGGAG GAGGGTC 17

(2) INFORMATION FOR SEQ ID NO: 26:

(i) SEQUENCE CHARACTERISTICS:

(A) LENGTH: 17
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 2 of HLA-A gene

(xi) SEQUENCE DESCRIPTION: SEQ ID NO:26:

GTCGTGACCT GCGCCCC 17

(2) INFORMATION FOR SEQ ID NO: 27:

(i) SEQUENCE CHARACTERISTICS:

(A) LENGTH: 19
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 3 of HLA-A gene

(xi) SEQUENCE DESCRIPTION: SEQ ID NO:27:

GGGCGGGGCG GGGCTCGGG 19

(2) INFORMATION FOR SEQ ID NO: 28:

(i) SEQUENCE CHARACTERISTICS:

(A) LENGTH: 23
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 3 of HLA-A gene

(xi) SEQUENCE DESCRIPTION: SEQ ID NO:28:

CGGGAGATCT ACAGGCGATC AGG 23

(2) INFORMATION FOR SEQ ID NO: 29:

(i) SEQUENCE CHARACTERISTICS:

(A) LENGTH: 18
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 2 of HLA-B gene

(xi) SEQUENCE DESCRIPTION: SEQ ID NO:29:

TCCCACTCCA TGAGGTAT 18

(2) INFORMATION FOR SEQ ID NO: 30:

(i) SEQUENCE CHARACTERISTICS:

(A) LENGTH: 17
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 2 of HLA-B gene

(xi) SEQUENCE DESCRIPTION: SEQ ID NO:30:

GTCGTGACCT GCGCCCC 17

(2) INFORMATION FOR SEQ ID NO: 31:

(i) SEQUENCE CHARACTERISTICS:

(A) LENGTH: 19
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 3 of HLA-B gene

(xi) SEQUENCE DESCRIPTION: SEQ ID NO:31:

GGGCGGGGCG GGGCTCGGG 19

(2) INFORMATION FOR SEQ ID NO: 32:

(i) SEQUENCE CHARACTERISTICS:

(A) LENGTH: 18
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM; human
(D) OTHER INFORMATION: primer for sequencing of exon 3 of HLA-B gene

(xi) SEQUENCE DESCRIPTION: SEQ ID NO:32:

GAAGGCTCCC CACTGCCC 18

(2) INFORMATION FOR SEQ ID NO: 33:

(i) SEQUENCE CHARACTERISTICS:

(A) LENGTH:18
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 2 of HLA-C gene

(xi) SEQUENCE DESCRIPTION: SEQ ID NO:33:

GGAGGGTCGG GCGGGTCT 18

(2) INFORMATION FOR SEQ ID NO: 34:

(i) SEQUENCE CHARACTERISTICS:

(A) LENGTH: 17
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 2 of HLA-C gene

(xi) SEQUENCE DESCRIPTION: SEQ ID NO:34:

GTCGTGACCT GCGCCCC 17

(2) INFORMATION FOR SEQ ID NO: 35:

(i) SEQUENCE CHARACTERISTICS:

(A) LENGTH: 19
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 3 of HLA-C gene

(xi) SEQUENCE DESCRIPTION: SEQ ID NO:35:

GACCGCGGGG GCGGGGCCA 19

(2) INFORMATION FOR SEQ ID NO: 36:

(i) SEQUENCE CHARACTERISTICS:

(A) LENGTH: 19
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: human
(D) OTHER INFORMATION: primer for sequencing of exon 3 of HLA-C gene

(xi) SEQUENCE DESCRIPTION: SEQ ID NO:36:

GACCACGGGG GCGGGGCCA 19

(2) INFORMATION FOR SEQ ID NO: 37:

(i) SEQUENCE CHARACTERISTICS:

(A) LENGTH: 17
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: human
(D) OTHER INFORMATION: primer for sequencing of exon 3 of HLA-C gene

(xi) SEQUENCE DESCRIPTION: SEQ ID NO:37:

GAGGCTCCCC ACTGCCC 17

(2) INFORMATION FOR SEQ ID NO: 38:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: Chlamydia trachomatis
(D) OTHER INFORMATION: primer for sequencing of cryptic plasmis

(xi) SEQUENCE DESCRIPTION: SEQ ID NO:38:

TCCGGAGCGA GTTACGAAGA 20

(2) INFORMATION FOR SEQ ID NO: 39:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Chlamydia trachomatis
(D) OTHER INFORMATION: primer for sequencing of cryptic plasmid

(xi) SEQUENCE DESCRIPTION: SEQ ID NO:39:

ATTCAATGCC CGGGATTGGT 20

(2) INFORMATION FOR SEQ ID NO: 40:

(i) SEQUENCE CHARACTERISTICS:

(A) LENGTH: 25
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:40:

CCGACCGCGT CTTGAAAACA GATGT 25

(2) INFORMATION FOR SEQ ID NO: 41:

(i) SEQUENCE CHARACTERISTICS:

(A) LENGTH: 21
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:41:

CACCCACATT CCCAGAGAGC T 41

(2) INFORMATION FOR SEQ ID NO: 42:

(i) SEQUENCE CHARACTERISTICS:

(A) LENGTH: 21
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEO ID NO:42:

CGTGCAGCTT TGTGGGAATG T 21

(2) INFORMATION FOR SEQ ID NO: 43:

(i) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:43:

CTAGATTTCA TCTTGTTCAA TTGC 24

(2) INFORMATION FOR SEQ ID NO: 44:

(i) SEQUENCE CHARACTERISTICS:

(A) LENGTH: 23
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:44:

AGCATGCGTR TKGGTTACTA YGG 23

(2) INFORMATION FOR SEQ ID NO: 45:

(i) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:45:

TGACTTTGTT TTCGACCGYG TTTT 24

(2) INFORMATION FOR SEQ ID NO: 46:

(i) SEQUENCE CHARACTERISTICS:

(A) LENGTH: 23
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:46:

CTAAAGTYGC RCATCCACAT TCC 23

(2) INFORMATION FOR SEQ ID NO: 47:

(i) SEQUENCE CHARACTERISTICS:

(A) LENGTH: 23
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:47:

CATCCACATT CCCASARAGC TGC 23

(2) INFORMATION FOR SEQ ID NO: 48:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Chlamydia trachomatis
(D) OTHER INFORMATION: primer for sequencing of cryptic plasmid

(xi) SEQUENCE DESCRIPTION: SEQ ID NO:48:

ATGCCCGGGA TTGGTTGATC

(2) INFORMATION FOR SEQ ID NO: 49:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:49:

GGAGACTTTG TTTTCGACCG 20 [SEQ ID NO 49]

(2) INFORMATION FOR SEQ ID NO: 50:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:50:

CATTCCCACA AAGCTGCGCG 20

(2) INFORMATION FOR SEQ ID NO: 51:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:51:

TTCCCACAAA GCTGCGCGAG 20

(2) INFORMATION FOR SEQ ID NO: 52:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Chlamydia trachomatis
(D) OTHER INFORMATION: primer for sequencing of VS regions

(xi) SEQUENCE DESCRIPTION: SEQ ID NO:52:

CCCACAAAGC TGCGCGAGCG 20

(2) INFORMATION FOR SEQ ID NO: 53:

(i) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: Chlamydia trachomatis
(D) OTHER INFORMATION: primer for sequencing of ribosomal DNA

(xi) SEQUENCE DESCRIPTION: SEQ ID NO:53:

ACCTTTCGGT TGAGGGAGAG TCTA 24

(2) INFORMATION FOR SEQ ID NO: 54:

(i) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Chlamydia trachomatis
(D) OTHER INFORMATION: primer for sequencing of ribosomal DNA

(xi) SEQUENCE DESCRIPTION: SEQ ID NO:54:

GGACCAATTC TTATTCCCAA GCGA 24

(2) INFORMATION FOR SEQ ID NO: 55:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: HIV-1
(D) OTHER INFORMATION: primer for sequencing of HIV-1 protease gene

(xi) SEQUENCE DESCRIPTION: SEQ ID NO:55:

ATCACTCTTT GGCAACGACC 20

(2) INFORMATION FOR SEQ ID NO: 56:

(i) SEQUENCE CHARACTERISTICS:

(A) LENGTH: 24
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: HIV-1
(D) OTHER INFORMATION: primer for sequencing of HIV-1 protease gene

(xi) SEQUENCE DESCRIPTION: SEQ ID NO:56:

CAGGAGCAGA TGATACAGTA TTAG 24

(2) INFORMATION FOR SEQ ID NO: 57:

(i) SEQUENCE CHARACTERISTICS:

(A) LENGTH: 25
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: HIV-1
(D) OTHER INFORMATION: primer for sequencing of HIV-1 protease gene

(xi) SEQUENCE DESCRIPTION: SEQ ID NO:57:

CCATTCCTGG CTTTAATTTT ACTGG 25

(2) INFORMATION FOR SEQ ID NO: 58:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: yes
(v) FRAGMENT TYPE: internal
(vi) ORIGINAL SOURCE:

(A) ORGANISM: Human Papillomavirus
(D) OTHER INFORMATION: primer for sequencing of HPV

(xi) SEQUENCE DESCRIPTION: SEQ ID NO:58:

GCMCAGGGWC ATAAYAATGG 20

(2) INFORMATION FOR SEQ ID NO: 59:

(i) SEQUENCE CHARACTERISTICS:

(A) LENGTH: 20
(B) TYPE: nucleic acid
(C) STRANDEDNESS: double
(D) TOPOLOGY: linear

(ii) MOLECULE TYPE: other nucleic acid
(iii) HYPOTHETICAL:no
(iv) ANTI-SENSE: no
(v) FRAGMENT TYPE: internal
(vi) ORIGINAL SOURCE:

(A) ORGANISM: Human Papillomavirus
(D) OTHER INFORMATION: primer for sequencing of HPV

(xi) SEQUENCE DESCRIPTION: SEQ ID NO:59:

CGTCCMAARG GAWACTGATC 20